# EUROPEAN PATENT SPECIFICATION

(11) **EP 1 487 485 B1**
(45) Date of publication and mention of the grant of the patent: **15.12.2010**
(21) Application number: 03712390.8
(22) Date of filing: 19.03.2003
(51) Int. Cl.: A61K 39/39, A61P 37/04

(54) **IMIDAZOQUINOLINE ADJUVANTS FOR DNA VACCINES**
ADJUVANTIEN FÜR DNS IMPSTOFFE BASIEREND AUF IMIDAZOCHINOLINE
IMIDAZOQUINOLINE ADJUVANT POUR VACCINS ADN

(30) Priority: 19.03.2002 US 102615; 19.03.2002 US 366057 P
(43) Date of publication of application: 22.12.2004
(73) Proprietor: PowderJect Research Limited, Sandwich, Kent CT13 9NJ (GB)
(72) Inventor: BRAUN, Ralph, Patrick, Middleton, WI 53562 (US)
(74) Representative: Woods, Geoffrey Corlett
(86) International application number: PCT/GB2003/001203
(87) International publication number: WO 2003/080114

(56) References cited:
- WO-A-02/24225
- WO-A-93/20847
- BURNS R P ET AL: "The imidazoquinolines, imiquimod and R-848, induced functional, but not phenotypic, maturation of human epidermal langerhans' cells" CLINICAL IMMUNOLOGY, ACADEMIC PRESS,, US, vol. 94, no. 1, January 2000 (2000-01), pages 13-23, XP002964628 ISSN: 1521-6616
- DOCKRELL D H ET AL: "IMIQUIMOD AND RESIQUIMOD AS NOVEL IMMUNOMODULATORS" JOURNAL OF ANTIMICROBIAL CHEMOTHERAPY, SAUNDERS CO. LTD., LONDON, GB, vol. 48, no. 6, December 2001 (2001-12), pages 751-755, XP001037921 ISSN: 0305-7453
- JOHNSON A G: "MOLECULAR ADJUVANTS AND IMMUNOMODULATORS: NEW APPROACHES TO IMMUNIZATION" CLINICAL MICROBIOLOGY REVIEWS, WASHINGTON, DC, US, vol. 7, no. 3, July 1994 (1994-07), pages 277-289, XP000942982 ISSN: 0893-8512
- Bernstein, D.I. and C.J. Harrison (1989) Antimicrob. Agents Chemother. 33:1511-1515
- Bernstein, D.I. et al (1993) J. Infect. Dis. 167:731-735
- Heath, A.W. et al (1989) Immunology 67:520-524
- Holland, G.P. et al (1990) Clin. exp. Immunol. 82:221-226
- Playfair, J.H.L. and J.B. Souza (1987) Clin.exp. Immunol. 67:5-10
- Pighetti, G.M. et al (1996) Am. J. Vet. Res. 57:819-824
- Xlang, Z. and H.C.J. Ertl (1995) Immunity 2:129-135

## Description

### Field of the invention

The invention relates to the fields of vaccines, vaccine adjuvants, molecular biology and immunology, and generally relates to adjuvants and nucleic acid immunization techniques. More specifically, the invention relates to certain adjuvant compositions, and to vaccine and/or nucleic acid immunization strategies employing such compositions.

### Background of the invention

Techniques for the injection of DNA and mRNA into mammalian tissue for the purposes of immunization against an expression product have been described in the art. The techniques, termed "nucleic acid immunization" herein, have been shown to elicit both humoral and cell-mediated immune responses. For example, sera from mice immunized with a DNA construct encoding the envelope glycoprotein, gp160, were shown to react with recombinant gp160 in immunoassays, and lymphocytes from the injected mice were shown to proliferate in response to recombinant gp120. Wang et al. (1993) Proc. Natl. Acad. Sci. USA 90:4156-4160.

Similarly, mice immunized with a human growth hormone (hGH) gene demonstrated an antibody-based immune response. Tang et al. (1992) Nature 356:152-154. Intramuscular injection of DNA encoding influenza nucleoprotein driven by a mammalian promoter has been shown to elicit a CD8+ CTL response that can protect mice against subsequent lethal challenge with virus. Ulmer et al. (1993) Science 259:1745-1749. Immunohistochemical studies of the injection site revealed that the DNA was taken up by myeloblasts, and cytoplasmic production of viral protein could be demonstrated for at least 6 months.

WO 93/20847 relates to immunogen/vaccine adjuvant compositions comprising an immunogen and as a vaccine adjuvant a 1H-imidazo[4,5-c]quinolin-4-amine. Suitable immunogens include live viral and bacterial immunogens and inactivated viral, tumor-derived, protozoal, organism-derived, fungal and bacterial immunogens, toxoids, toxins polysaccharides, proteins, glycoproteins, peptides, and the like.

Clin. Microbiol. Rev. 7:277-289 (1994) reviews the use of the adjuvant imiquimod on a herpes simplex virus type 2 glycoprotein vaccine in guinea pigs.

### Summary of the invention

The inventors have found that an imidazo quinoline amine compound acts as an effective adjuvant when administered topically 12 to 36 hours after a primer or booster immunisation. In addition the compound was found to be effective in stimulating cell-mediated immunity. The compound is from among a series of related compounds known to be capable of modifying immune responses.

Accordingly, the invention provides a compound for use in a method for enhancing or stimulating in an individual a cell-mediated immune response to an antigen, wherein the compound is an imidazoquinoline amine, imidazopyridine amine, 6,7-fused cycloalkylimidazopyridine amine, 1,2-bridged imidazoquinoline amine, thiazolo- or oxazolo-quinolinamine, thiazolo- or oxazolo-pyridinamine, imidazonaphthyridine or tetrahydroimidazonaphthyridine amine, and wherein the compound is administered topically or transdermally to the individual 12 to 36 hours after an isolated recombinant polynucleotide capable of expressing the antigen is administered.

The invention also provides an isolated recombinant polynucleotide capable of expressing an antigen for use in a method for enhancing or stimulating in an individual a cell-mediated immune response to said antigen, wherein the polynucleotide is administered 12 to 36 hours before topical or transdermal administration to the individual of a compound that is an imidazoquinoline amine, imidazopyridine amine, 6,7-fused cycloalkylimidazopyridine amine, 1,2-bridged imidazoquinoline amine, thiazolo- or oxazolo-quinolinamine, thiazolo- or oxazolo-pyridinamine, imidazonaphthyridine or tetrahydroimidazonaphthyridine amine.

Furthermore, the invention provides the use of a compound in the manufacture of a medicament for enhancing or stimulating in an individual a cell-mediated immune response to an antigen, wherein the compound is an imidazoquinoline amine, imidazopyridine amine, 6,7-fused cycloalkylimidazopyridine amine, 1,2-bridged imidazoquinoline amine, thiazolo- or oxazolo-quinolinamine, thiazolo- or oxazolo-pyridinamine, imidazonaphthyridine or tetrahydroimidazonaphthyridine amine, and wherein the compound is for administration topically or transdermally to the individual 12 to 36 hours after an isolated recombinant polynucleotide capable of expressing the antigen is administered.

### Brief description of the Figures

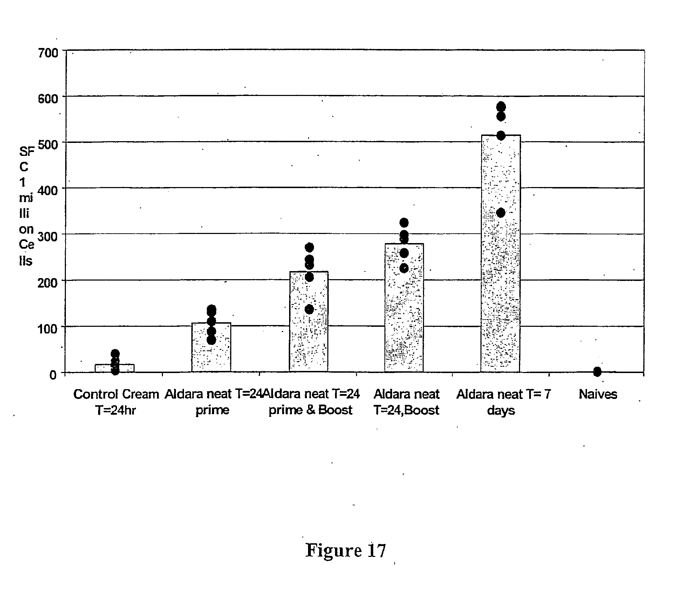

Figures 1 to 17 show the adjuvant activity of the compound of the invention in a series of vaccination experiments.
Figure 16 shows IFN-γ sAg Elispots 2 weeks post prime when imiquimod is used as adjuvant with pdpsc18.1. The administrations for columns 1 to 13 of the Figure are as follows:
   1. 2.0 ug pdpsc 18
   2. 0.2 ug pdpsc 18
   3. 0.02 ug pdpsc 18
   4. 2.0 ug pdpsc 18 IMQ before
   5. 0.2 ug pdpsc 18 IMQ before
   6. 0.02 ug pdpsc 18 IMQ before
   7. 2.0 ug pdpsc 18 IMQ right after
   8. 0.2 ug pdpsc 18 IMQ right after
   9. 0.02 ug pdpsc 18 IMQ right after
   10. 2.0 ug pdpsc 18 IMQ 24 hr pp
   11. 0.2 ug pdpsc 18 IMQ 24 hr pp
   12. 0.02 ug pdpsc 18 IMQ 24 hr pp
   13. 2 ug p7313p1c
Figure 17 shows IFN-γ Elispots for HBV sAg peptide.

### Detailed description of the invention

It is to be understood that this invention is not limited to particular antigens or to antigen-coding nucleotide sequences. It is also to be understood that different applications of the disclosed methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "an antigen" includes a mixture of two or more such agents, reference to "a particle" includes reference to mixtures of two or more particles, reference to "a recipient cell" includes two or more such cells, and the like.

The invention provides for enhancing an immune response to an antigen. The compound is administered topically or transdermally to an individual 12 to 36 hours after administration of an isolated recombinant polynucleotide capable of expressing an antigen. Typically, the compound is administered 16 to 32, 20 to 28 or preferably 22 to 26 hours after administration of polynucleotide. Thus the compound may be administered about 24 hours after administration of the polynucleotide.

The administration of the polynucleotide (after which the compound is administered) may be one of a series of administrations of polynucleotide or antigen which occur in an administration regimen. Therefore, the administration of the polynucleotide may be a priming or boosting administration. Thus, in one embodiment of the invention the compound is administered 12 to 36 hours after a priming administration and/or 12 to 36 hours after a booster administration.

In one embodiment, nothing further is administered to the individual in the 12 to 36 hour period (or any of the other time periods mentioned above) between administration of the polynucleotide and the administration of the compound. Alternatively, in some embodiments, in this time period no polynucleotide is administered, or preferably at least not the same polynucleotide which was administered before. No further compound is administered in the time period.

The aim of the invention is to enhance the immune response to the antigen. Cell-mediated immunity is enhanced, and in particular the CD8 T cell response is enhanced. In this case the administration of the compound increases the level of CD8 T cell response, for example increases the level of antigen experienced CD8 T cells. The increase in the CD8 T cell response may be measured using any suitable assay (and thus may be capable of being detected in such an assay), such as an ELISPOT assay, preferably an IFN-γ ELISPOT assay.

In one embodiment, the CD4 T cell response is also enhanced, such as the CD4 Th1 response. Thus, the levels of antigen-experienced CD4 T cells may also be increased. Such increased levels of CD4 T cells may be detected using a suitable assay, such as a proliferation assay.

Administration of the compound may cause the immune response to shift to a cell-mediated response. Thus, the immune response may shift towards a Th1 response and/or the ratio of IgG1 to IgG2a antibody may decrease. In one embodiment, the administration of the compound causes a decrease in antibody response.

The compound is selected from an imidazoquinoline amine, imidazopyridine amine, 6,7-fused cycloalkylimidazopyridine amine, 1,2-bridged imidazoquinoline amine, thiazolo- and oxazolo-quinolinamines and pyridinamines, imidazonaphthyridine and tetrahydroimidazonaphthyridine amine.

Preferred compounds include 1H-imidazo[4,5-c]quinolin-4-amines defined by one of Formulas I-V below: 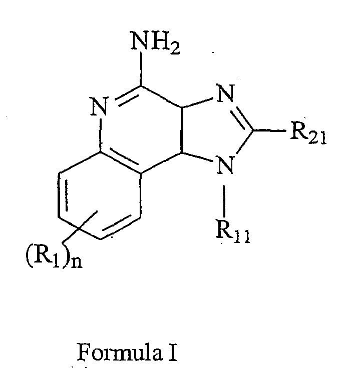 wherein:
R11 is selected from the group consisting of alkyl of one to ten carbon atoms, hydroxyalkyl of one to six carbon atoms, acyloxyalkyl wherein the acyloxy moiety is alkanoyloxy of two to four carbon atoms or benzoyloxy, and the alkyl moiety contains one to six carbon atoms, benzyl, (phenyl)ethyl and phenyl, said benzyl, (phenyl)ethyl or phenyl substituent being optionally substituted on the benzene ring by one or two moieties independently selected from the group consisting of alkyl of one to four carbon atoms, alkoxy of one to four carbon atoms and halogen, with the proviso that if said benzene ring is substituted by two of said moieties, then said moieties together contain no more than six carbon atoms;
R21 is selected from the group consisting of hydrogen, alkyl of one to eight carbon atoms, benzyl, (phenyl)ethyl and phenyl, the benzyl, (phenyl)ethyl or phenyl substituent being optionally substituted on the benzene ring by one or two moieties independently selected from the group consisting of alkyl of one to four carbon atoms, alkoxy of one to four carbon atoms and halogen, with the proviso that when the benzene ring is substituted by two of said moieties, then the moieties together contain no more than six carbon atoms; and
each R1 is independently selected from the group consisting of alkoxy of one to four carbon atoms, halogen, and alkyl of one to four carbon atoms, and n is an integer from 0 to 2, with the proviso that if n is 2, then said R1 groups together contain no more than six carbon atoms; 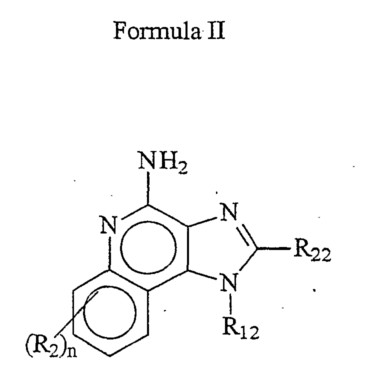 wherein:
R12 is selected from the group consisting of straight chain or branched chain alkenyl containing two to ten carbon atoms and substituted straight chain or branched chain alkenyl containing two to ten carbon atoms, wherein the substituent is selected from the group consisting of straight chain or branched chain alkyl containing one to four carbon atoms and cycloalkyl containing three to six carbon atoms; and cycloalkyl containing three to six carbon atoms substituted by straight chain or branched chain alkyl containing one to four carbon atoms; and
R22 is selected from the group consisting of hydrogen, straight chain or branched chain alkyl containing one to eight carbon atoms, benzyl, (phenyl)ethyl and phenyl, the benzyl, (phenyl)ethyl or phenyl substituent being optionally substituted on the benzene ring by one or two moieties independently selected from the group consisting of straight chain or branched chain alkyl containing one to four carbon atoms, straight chain or branched chain alkoxy containing one to four carbon atoms, and halogen, with the proviso that when the benzene ring is substituted by two such moieties, then the moieties together contain no more than six carbon atoms; and
each R2 is independently selected from the group consisting of straight chain or branched chain alkoxy containing one to four carbon atoms, halogen, and straight chain or branched chain alkyl containing one to four carbon atoms, and n is an integer from zero to 2, with the proviso that if n is 2, then said R2 groups together contain no more than six carbon atoms; 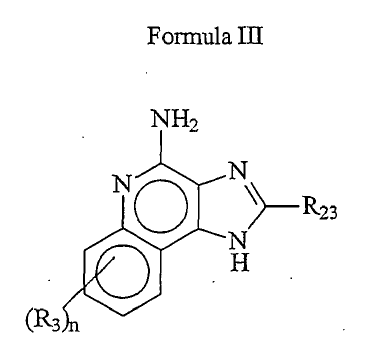 wherein:
R23 is selected from the group consisting of hydrogen, straight chain or branched chain alkyl of one to eight carbon atoms, benzyl, (phenyl)ethyl and phenyl, the benzyl, (phenyl)ethyl or phenyl substituent being optionally substituted on the benzene ring by one or two moieties independently selected from the group consisting of straight chain or branched chain alkyl of one to four carbon atoms, straight chain or branched chain alkoxy of one to four carbon atoms, and halogen, with the proviso that when the benzene ring is substituted by two such moieties, then the moieties together contain no more than six carbon atoms; and
each R3 is independently selected from the group consisting of straight chain or branched chain alkoxy of one to four carbon atoms, halogen, and straight chain or branched chain alkyl of one to four carbon atoms, and n is an integer from zero to 2, with the proviso that if n is 2, then said R3 groups together contain no more than six carbon atoms; 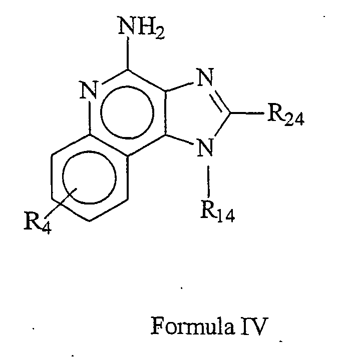 wherein:
R14 is -CHRₓR_{y} wherein R_{y} is hydrogen or a carbon-carbon bond, with the proviso that when R_{y} is hydrogen Rₓ is alkoxy of one to four carbon atoms, hydroxyalkoxy of one to four carbon atoms, 1-alkynyl of two to ten carbon atoms, tetrahydropyranyl, alkoxyalkyl wherein the alkoxy moiety contains one to four carbon atoms and the alkyl moiety contains one to four carbon atoms, 2-, 3-, or 4-pyridyl, and with the further proviso that when R_{y} is a carbon-carbon bond R_{y} and Rₓ together form a tetrahydrofuranyl group optionally substituted with one or more substituents independently selected from the group consisting of hydroxy and hydroxyalkyl of one to four carbon atoms;
R24 is selected from the group consisting of hydrogen, alkyl of one to four carbon atoms, phenyl, and substituted phenyl wherein the substituent is selected from the group consisting of alkyl of one to four carbon atoms, alkoxy of one to four carbon atoms, and halogen; and
R4 is selected from the group consisting of hydrogen, straight chain or branched chain alkoxy containing one to four carbon atoms, halogen, and straight chain or branched chain alkyl containing one to four carbon atoms; 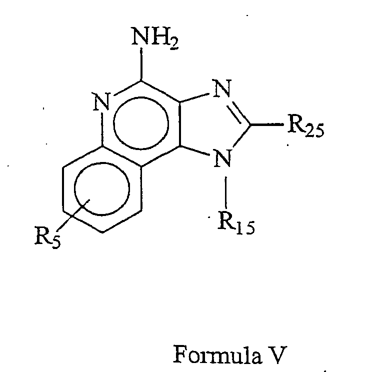 wherein:
R15 is selected from the group consisting of: hydrogen; straight chain or branched chain alkyl containing one to ten carbon atoms and substituted straight chain or branched chain alkyl containing one to ten carbon atoms, wherein the substituent is selected from the group consisting of cycloalkyl containing three to six carbon atoms and cycloalkyl containing three to six carbon atoms substituted by straight chain or branched chain alkyl containing one to four carbon atoms; straight chain or branched chain alkenyl containing two to ten carbon atoms and substituted straight chain or branched chain alkenyl containing two to ten carbon atoms, wherein the substituent is selected from the group consisting of cycloalkyl containing three to six carbon atoms and cycloalkyl containing three to six carbon atoms substituted by straight chain or branched chain alkyl containing one to four carbon atoms; hydroxyalkyl of one to six carbon atoms; alkoxyalkyl wherein the alkoxy moiety contains one to four carbon atoms and the alkyl moiety contains one to six carbon atoms; acyloxyalkyl wherein the acyloxy moiety is alkanoyloxy of two to four carbon atoms or benzoyloxy, and the alkyl moiety contains one to six carbon atoms; benzyl; (phenyl)ethyl; and phenyl; said benzyl, (phenyl)ethyl or phenyl substituent being optionally substituted on the benzene ring by one or two moieties independently selected from the group consisting of alkyl of one to four carbon atoms, alkoxy of one to four carbon atoms, and halogen, with the proviso that when said benzene ring is substituted by two of said moieties, then the moieties together contain no more than six carbon atoms;
R15 is preferably a C1-2 alkyl group which is substituted by a hydroxyalkyl of 1 to 4 carbon atoms, and more preferably R15 is a C1 alkyl group which is substituted by a hydroxyalkyl of 3 carbon atoms;
R25 sis 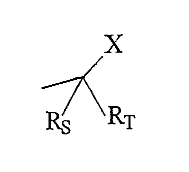 wherein:
R_{S} and R_{T} are independently selected from the group consisting of hydrogen, alkyl of one to four carbon atoms, phenyl, and substituted phenyl wherein the substituent is selected from the group consisting of alkyl of one to four carbon atoms, alkoxy of one to four carbon atoms, and halogen;
X is selected from the group consisting of alkoxy containing one to four carbon atoms, alkoxyalkyl wherein the alkoxy moiety contains one to four carbon atoms and the alkyl moiety contains one to four carbon atoms, hydroxyalkyl of one to four carbon atoms, haloalkyl of one to four carbon atoms, alkylamido wherein the alkyl group contains one to four carbon atoms, amino, substituted amino wherein the substituent is alkyl or hydroxyalkyl of one to four carbon atoms, azido, chloro, hydroxy, 1-morpholino, 1-pyrrolidino, alkylthio of one to four carbon atoms; and
R5 is selected from the group consisting of hydrogen, straight chain or branched chain alkoxy containing one to four carbon atoms, halogen, and straight chain or branched chain alkyl containing one to four carbon atoms;
or a pharmaceutically acceptable salt of any of the foregoing.

Preferred 6,7 fused cycloalkylimidazopyridine amine compounds are defined by Formula VI below: 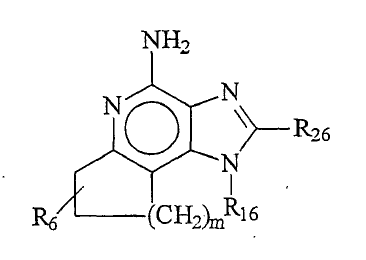 wherein m is 1, 2, or 3;
R16 is selected from the group consisting of hydrogen; cyclic alkyl of three, four, or five carbon atoms; straight chain or branched chain alkyl containing one to ten carbon atoms and substituted straight chain or branched chain alkyl containing one to ten carbon atoms, wherein the substituent is selected from the group consisting of cycloalkyl containing three to six carbon atoms and cycloalkyl containing three to six carbon atoms substituted by straight chain or branched chain alkyl containing one to four carbon atoms; fluoro- or chloroalkyl containing from one to ten carbon atoms and one or more fluorine or chlorine atoms; straight chain or branched chain alkenyl containing two to ten carbon atoms and substituted straight chain or branched chain alkenyl containing two to ten carbon atoms, wherein the substituent is selected from the group consisting of cycloalkyl containing three to six carbon atoms and cycloalkyl containing three to six carbon atoms substituted by straight chain or branched chain alkyl containing one to four carbon atoms; hydroxyalkyl of one to six carbon atoms; alkoxyalkyl wherein the alkoxy moiety contains one to four carbon atoms and the allyl moiety contains one to six carbon atoms; acyloxyalkyl wherein the acyloxy moiety is alkanoyloxy of two to four carbon atoms or benzoyloxy, and the alkyl moiety contains one to six carbon atoms, with the proviso that any such alkyl, substituted alkyl, alkenyl, substituted alkenyl, hydroxyalkyl, alkoxyalkyl, or acyloxyalkyl group does not have a fully carbon substituted carbon atom bonded directly to the nitrogen atom; benzyl; (phenyl)ethyl; and phenyl; said benzyl, (phenyl)ethyl or phenyl substituent being optionally substituted on the benzene ring by one or two moieties independently selected from the group consisting of alkyl of one to four carbon atoms, alkoxy of one to four carbon atoms, and halogen, with the proviso that when said benzene ring is substituted by two of said moieties, then the moieties together contain no more than six carbon atoms;
and -CHRₓR_{y}
wherein:
R_{y} is hydrogen or a carbon-carbon bond, with the proviso that when R_{y} is hydrogen Rₓ is alkoxy of one to four carbon atoms, hydroxyalkoxy of one to four carbon atoms, 1-alkynyl of two to ten carbon atoms, tetrahydropyranyl, alkoxyalkyl wherein the alkoxy moiety contains one to four carbon atoms and the alkyl moiety contains one to four carbon atoms, 2-, 3-, or 4-pyridyl, and with the further proviso that when R_{y} is a carbon-carbon bond R_{y} and Rₓ together form a tetrahydrofuranyl group optionally substituted with one or more substituents independently selected from the group consisting of hydroxy and hydroxyalkyl of one to four carbon atoms,
R26 is selected from the group consisting of hydrogen, straight chain or branched chain alkyl containing one to eight carbon atoms, straight chain or branched chain hydroxyalkyl containing one to six carbon atoms, morpholinoalkyl, benzyl, (phenyl)ethyl and phenyl, the benzyl, (phenyl)ethyl or phenyl substituent being optionally substituted on the benzene ring by a moiety selected from the group consisting of methyl, methoxy, and halogen; and
-C(R_{S})(R_{T})(X) wherein R_{S} and R_{T} are independently selected from the group consisting of hydrogen, alkyl of one to four carbon atoms, phenyl, and substituted phenyl wherein the substituent is selected from the group consisting of alkyl of one to four carbon atoms, alkoxy of one to four carbon atoms, and halogen;
X is selected from the group consisting of alkoxy containing one to four carbon atoms, alkoxyalkyl wherein the alkoxy moiety contains one to four carbon atoms and the alkyl moiety contains one to four carbon atoms, haloalkyl of one to four carbon atoms, alkylamido wherein the alkyl group contains one to four carbon atoms, amino, substituted amino wherein the substituent is alkyl or hydroxyalkyl of one to four carbon atoms, azido, alkylthio of one to four carbon atoms, and morpholinoalkyl wherein the alkyl moiety contains one to four carbon atoms, and
R6 is selected from the group consisting of hydrogen, fluoro, chloro, straight chain or branched chain alkyl containing one to four carbon atoms, and straight chain or branched chain fluoro- or chloroalkyl containing one to four carbon atoms and at least one fluorine or chlorine atom;
and pharmaceutically acceptable salts thereof.

Preferred imidazopyridine amine compounds are defined by Formula VII below: 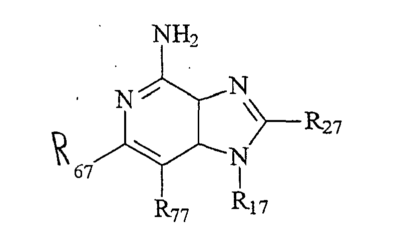 wherein
R17 is selected from the group consisting of hydrogen; -CH₂R_{W} wherein R_{W} is selected from the group consisting of straight chain, branched chain, or cyclic alkyl containing one to ten carbon atoms, straight chain or branched chain alkenyl containing two to ten carbon atoms, straight chain or branched chain hydroxyalkyl containing one to six carbon atoms, alkoxyalkyl wherein the alkoxy moiety contains one to four carbon atoms and the alkyl moiety contains one to six carbon atoms, and phenylethyl; and -CH=CR_{Z}R_{Z} wherein each R_{Z} is independently straight chain, branched chain, or cyclic alkyl of one to six carbon atoms;
R27 is selected from the group consisting of hydrogen, straight chain or branched chain alkyl containing one to eight carbon atoms, straight chain or branched chain hydroxyalkyl containing one to six carbon atoms, alkoxyalkyl wherein the alkoxy moiety contains one to four carbon atoms and the alkyl, moiety contains one to six carbon atoms, benzyl, (phenyl)ethyl and phenyl, the benzyl, (phenyl)ethyl or phenyl substituent being optionally substituted on the benzene ring by a moiety selected from the group consisting of methyl, methoxy, and halogen; and morpholinoalkyl wherein the alkyl moiety contains one to four carbon atoms;
R67 and R77 are independently selected from the group consisting of hydrogen and alkyl of one to five carbon atoms, with the proviso that R67 and R77 taken together contain no more than six carbon atoms, and with the further proviso that when R77 is hydrogen then R67 is other than hydrogen and R27 is other than hydrogen or morpholinoalkyl, and with the further proviso that when R67 is hydrogen then R77 and R27 are other than hydrogen;
and pharmaceutically acceptable salts thereof.

Preferred 1,2-bridged imidazoquinoline amine compounds are defined by Formula VIII below: 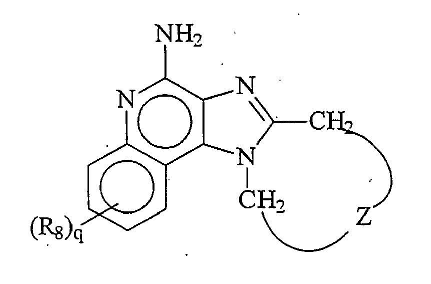 wherein:
Z is selected from the group consisting of:
   -(CH₂)p- wherein p is 1 to 4;
   -(CH₂)a -C(R_{D}R_{B})(CH₂)_{b}-, wherein a and b are integers and a+b is 0 to 3, R_{D} is hydrogen or alkyl of one to four carbon atoms, and R_{E} is selected from the group consisting of alkyl of one to four carbon atoms, hydroxy, -OR_{F} wherein R_{F} is alkyl of one to four carbon atoms, and -NR_{G} R'_{G} wherein R_{G} and R'_{G} are independently hydrogen or alkyl of one to four carbon atoms; and -(CH₂)ₐ -(Y)-(CH₂)_{b}- wherein a and b are integers and a+b is 0 to 3, and Y is O, S, or - NR_{J} wherein R_{J} is hydrogen or alkyl of one to four carbon atoms;
      and wherein q is 0 or 1 and R8 is selected from the group consisting of alkyl of one to four carbon atoms, alkoxy of one to four carbon atoms, and halogen,
      and pharmaceutically acceptable salts thereof.

Suitable thiazolo- and oxazolo-quinolinamine and pyridinamine compounds include compounds of Formula IX and Formula IXa: 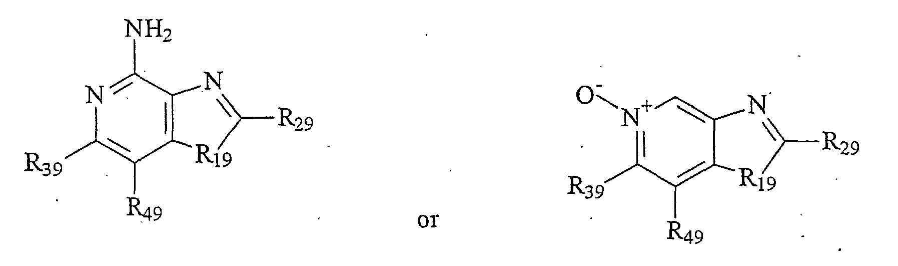 wherein:
R19 is selected from the group consisting of oxygen, sulfur and selenium;
R29 is selected from the group consisting of
   -hydrogen;
   -alkyl;
   -alkyl-OH;
   -haloalkyl-,
   -alkenyl;
   -alkyl-X-alkyl;
   -alkyl-X-alkenyl;
   -alkenyl-X-alkyl;
   -alkenyl-X-alkenyl;
   -alkyl-N(R59)₂ ;
   -allcyl-N₃ ;
   -alkyl-O-C(O)-N(R59)₂;
   -heterocyclyl;
   -alkyl-X-heterocyclyl;
   -alkenyl-X-heterocyclyl;
   -aryl;
   -alkyl-X-azyl;
   -alkenyl-X-alyl;
   -heteroaryl;
   -alkyl-X-heteroaryl; and
   -alkenyl-X-heteroaryl;
R39 and R49 are each independently:
   -hydrogen;
   -X-alkyl;
   -halo;
   -haloalkyl;
   -N(R59)₂;
      or when taken together, R39 and R49 form a fused aromatic, heteroaromatic, cycloalkyl or heterocyclic ring;.
X is selected from the group consisting of -O-, -S-, -NR59-, -C(O)-, -C(O)O-, -OC(O)-, and a bond; and
each R59 is independently H or C₁₋₈ alkyl;

For formula IX and IXa the terms "alkyl" and "alkenyl" refer to a straight or branched hydrocarbon group, or a cyclic group (i.e., cycloalkyl and cycloalkenyl) that contains from 1 to 20, preferably 1 to 10, more preferably 1 to 8 carbon atoms, unless otherwise specified. Typical alkyl groups are, for example, methyl, ethyl, n-propyl, isopropyl, n-butyl, isobutyl, sec-butyl, tert-butyl, n-pentyl, n-hexyl, n-heptyl, n-octyl, and the like. Exemplary cyclic groups include cyclopropyl, cyclopentyl, cyclohexyl, cyclohexenyl and adamantyl. The prefix "alk," when used, e.g. for "alkoxy" and the like, also has the same meaning.

The term "aryl" refers to a carbocyclic aromatic ring or ring system. The aryl group is preferably a six-membered ring, such as phenyl, or an aromatic polycyclic ring system, such as naphthyl. The most preferred aryl group is phenyl which may be unsubstituted or substituted by one or more substituents as defined below. Examples of other suitable aryl groups include biphenyl, fluorenyl and indenyl.

The term "heteroaryl" refers to an aromatic ring or ring system that contains one or more heteroatoms, in which the beteroatoms are selected from nitrogen, oxygen and sulfur. Suitable heteroaryl groups include furyl, thienyl, pyridyl, quinolinyl, tetrazolyl, imidazo, and so on. In the case where R3 and R4 are taken together and form a 5- or 6-membered heteroaromatic ring, the heteroatom is nitrogen, oxygen or sulfur and the ring may contain one or more of such atoms. Preferably, the heteroatom is nitrogen or sulfur. Preferred heteroaromatic rings formed by R3 and R4 are illustrated by the following formulae where the two lines indicate where they are fused.

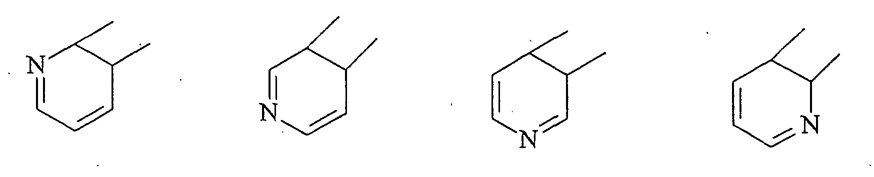

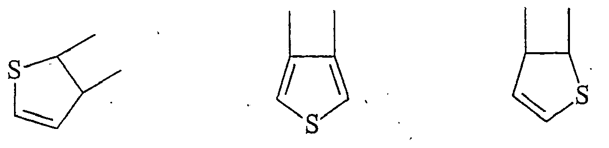

The terms "heterocyclic" and "heterocyclyl" refer to non-aromatic rings or ring systems that contain one or more ring heteroatoms (e.g., O, S, N). Exemplary heterocyclic groups include pyrrolidinyl, tetrahydrofuranyl, morpholinyl, piperidino, piperazino, thiazolidinyl, imidazolidinyl, and the like.

All of the above rings and ring systems can be unsubstituted or substituted by one or more substituents selected from the group consisting of alkyl, alkoxy, alkylthio, hydroxy, halogen, haloalkyl, polyhaloalkyl, perhaloalkyl (e.g., trifluoromethyl), trifluoroalkoxy (e.g., trifluoromethoxy), nitro, amino, alkylamino, dialkylamino, alkylcarbonyl, alkenylcaxbonyl, arylcarbonyl, heteroarylcarbonyl, aryl, arylakyl, heteroaryl, heteroarylalkyl, heterocyclyl, heterocycloalkyl, nitrile and alkoxycarbonyl. Preferred substituents are C₁₋₄ alkyl, C₁₋₄ alkoxy, halo, amino, alkylamino, dialk-ylamino, hydroxy, C₁₋₄ alkoxymethyl and trifluoromethyl.

The term "halo" refers to a halogen atom, such as, for example, fluorine, chlorine, bromine or iodine.

Suitable imidazonaphthyridine and tetrahydroimidazomaphthyridine compounds are those of Formulae X and XI below: 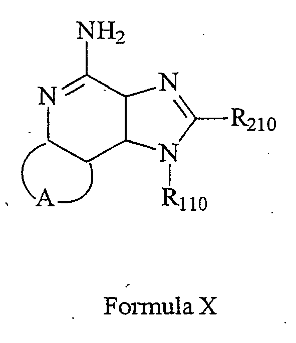 wherein
A is =N-CR=CR-CR=; =CR-N=CR-CR=; =CR-CR=N-CR=; or =CR-CR=CR-N=;

R110 is selected from the group consisting of:
- hydrogen;
- C₁₋₂₀ alkyl or C₂₋₂₀ alkenyl that is unsubstituted or substituted by one or more substituents selected from the group consisting of:
   -aryl;
   -heteroalyl;
   -heterocyclyl;
   -O-C₁₋₂₀ alkyl,
   -O-(C₁₋₂₀ alkyl)₀₋₁ -aryl;
   -O-(C₁₋₂₀ alkyl)₀₋₁ -heteroaryl;
   -O-(C₁₋₂₀ alkyl)₀₋₁ -heterocyclyl;
   -C₁₋₂₀ alkoxycarbonyl;
   -S(O)0-2-C₁₋₂₀ alkyl;
   -S(O)₀₋₂ -(C₁₋₂₀ alkl)₀₋₁ -aryl;
   -S(O)₀₋₂ -(C₁₋₂₀ alkyl)₀₋₁ -heteroaryl;
   -S(O)₀₋₂ -(C₁₋₂₀ alkyl)₀₋₁ -heterocyclyl;
   -N(R310)₂;
   -N₃;
   oxo;
   -halogen;
   -NO₂;
   -OH; and
   -SH; and
   -C₁₋₂₀ alkyl-NR310-Q-X-R410 or -C₂₋₂₀ alkenyl-NR310 -Q-X-R410 wherein Q is -CO-or -SO₂-; X is a bond, -O- or -NR310 - and R410 is aryl; heteroaryl; heterocyclyl; or - C₁₋₂₀ alkyl or C₂₋₂₀ alkenyl that is unsubstituted or substituted by one or more substituents selected from the group consisting of:
      -aryl;
      -heteroaryl;
      -heterocyclyl;
      -O-C₁₋₂₀ alkyl,
      -O-(C₁₋₂₀ alkyl)₀₋₁ -aryl;
      -O-(C₁₋₂₀ alkyl)₀₋₁ -heteroaryl;
      -O-(C₁₋₂₀ alkyl)₀₋₁ -heterocyclyl;
      -C₁₋₂₀ alkoxycarbonyl;
      -S(O)₀₋₂ -C₁₋₂₀ alkyl;
      -S(O)₀₋₂ -(C₁₋₂₀ alkyl)₀₋₁ -aryl;
      -S(O)₀₋₂ -(C₁₋₂₀ alkyl)₀₋₁ -heteroaryl;
      -S(O)₀₋₂ -(C₁₋₂₀ alky)₀₋₁ -heterocyclyl;
      -N(R310)₂;
      -NR310 -CO-O-C₁₋₂₀ alkyl;
      -N₃;
      oxo;
      -halogen;
      -NO₂;
      -OH; and
      -SH; or R410 is 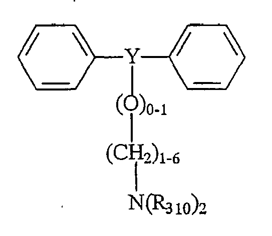
   wherein Y is -N- or -CR-;

R210 is selected from the group consisting of:
- hydrogen;
- C₁₋₁₀ alkyl;
- C₂₋₁₀ alkenyl;
- acryl;
- C₁₋₁₀ alkyl -O-C₁₋₁₀ -alkyl;
- C₁₋₁₀ alkyl-O-C₂₋₁₀ alkenyl; and
- C₁₋₁₀ alkyl orC₂₋₁₀ alkenyl substituted by one or more substituents selected from the group consisting of:
   - OH;
   - halogen;
   - N(R310)₂;
   - CO-N(R310)₂ ;
   - CO-C₁₋₁₀ alkyl;
   - N₃;
   - aryl;
   - heteroaryl;
   - heterocyclyl;
   - CO-aryl; and
   - CO-heteroaryl;
   each R310 is independently selected from the group consisting of hydrogen and C₁₋₁₀ alkyl; and
   each R is independently selected from the group consisting of hydrogen, C₁₋₁₀ alkyl, C₁₋₁₀ alkoxy, halogen and trifluoromethyl, or a pharmaceutically acceptable salt thereof. 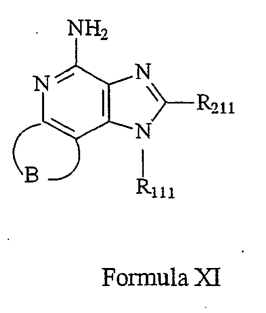 wherein:
   B is -NR-C(R)₂ -C(R)₂ -C(R)₂ -; -C(R)₂ -NR-C(R)₂ -C(R)₂ -; -C(R)₂ -C(R)₂-NR-C(R)₂ - or -C(R)₂ -C(R)₂ -C(R)₂ -NR-;

R111 is selected from the group consisting of:
- hydrogen;
- C₁₋₂₀ alkyl or C₂₋₂₀ alkenyl that is unsubstituted or substituted by one or more substituents selected from the group consisting of:
   - aryl;
   - heteroaryl;
   - heterocyclyl;
   - O-C₁₋₂₀ alkyl;
   - O-(C₁₋₂₀ alkyl)₀₋₁ -aryl;
   - O-(C₁₋₂₀ alkyl)₀₋₁ -heteroaryl;
   - O-(C₁₋₂₀ alkyl)₀₋₁ -heterocyclyl;
   - C₁₋₂₀ alkoxycarbonyl;
   - S(O)₀₋₂ -C₁₋₂₀ alkyl;
   - S(O)₀₋₂ -(C₁₋₂₀ alkyl)₀₋₁ -aryl;
   - S(O)₀₋₂ -(C₁₋₂₀ alkyl)₀₋₁ -heteroaryl;
   - S(O)₀₋₂ -(C₁₋₂₀ alkyl)₀₋₁ -heterocyclyl;
   - N(R311)₂;
   - N₃,
   - oxo;
   - halogen;
   - NO₂;
   - OH; and
   - SH; and
   - C₁₋₂₀ alkyl-NR311 -Q-X-R411 or -C₂₋₂₀ alkenyl-NR311 -Q-X-R411 wherein Q -CO-or -SO₂-; X is a bond, -O- or -NR311 - and R411 is aryl; heteroaryl- heterocyclyl; or - C₁₋₂₀ alkyl or C₂₋₂₀ alkenyl that is unsubstituted or substituted by one or more substituents selected from the group consisting of:
      -aryl;
      -heteroaryl;
      -heterocyclyl;
      -O-C₁₋₂₀ alkyl,
      -O-(C₁₋₂₀ alkyl)₀₋₁ -aryl;
      -O-(C₁₋₂₀ alkyl)₀₋₁ -heteroalyl;
      -O-(C₁₋₂₀ alkyl)₀₋₁ -heterocyclyl;
      -C₁₋₂₀ alkoxycarbonyl;
      -S(O)₀₋₂ -C₁₋₂₀ alkyl;
      -S(O)₀₋₂ -(C₁₋₂₀ alkyl)₀₋₁ -aryl;
      -S(O)₀₋₂ -(C₁₋₂₀ alkyl)₀₋₁ -heteroaryl;
      -S(O)₀₋₂ -(C₁₋₂₀ alkyl)₀₋₁ -heterocyclyl;
      -N(R311)₂ ;
      -NR311 -CO-O-C₁₋₂₀ alkyl;
      -N₃;
      oxo;
      -halogen;
      -NO₂ ;
      -OH; and
      -SH; or R411 is 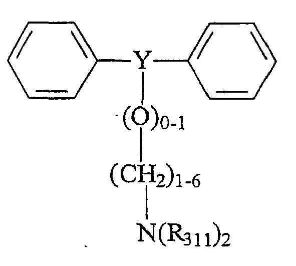
   wherein Y is -N- or -CR-;

R211 is selected from the group consisting of:
- hydrogen;
- C₁₋₁₀ alkyl;
- C₂₋₁₀ alkenyl;
- aryl
- C₁₋₁₀ alkyl-O-C₁₋₁₀ -alkyl;
- C₁₋₁₀ alkyl-O-C₂₋₁₀ alkenyl; and
- C₁₋₁₀ alkyl or C₂₋₁₀ alkenyl substituted by one or more substituents selected from the group consisting of:
   - OH;
   - halogen;
   - N(R311)₂ ;
   - CO-N(R311)₂ ;
   - CO-C1-10 alkyl;
   - N₃ ;
   - acryl;
   - heteroaryl;
   - heterocyclyl;
   - CO-aryl; and
   - CO-heteroaryl;
   each R 311 is independently selected from the group consisting of hydrogen and C₁₋₁₀ alkyl; and
   each R is independently selected from the group consisting of hydrogen, C₁₋₁₀ alkyl, C₁₋₁₀ alkoxy, halogen and trifluoromethyl, and pharmaceutically acceptable salts thereof.

The substituents R11 -R111 above are generally designated "1-substituents" herein. The preferred 1-substituents are alkyl containing one to six carbon atoms and hydroxyalkyl containing one to six carbon atoms. More preferably the 1- substituent is 2-methylpropyl or 2-hydroxy-2-methylpropyl.

The substituents R21 -R211 above are generally designated "2-substituents" herein. The preferred 2-substituents are hydrogen, alkyl of one to six carbon atoms, alkoxyalkyl wherein the alkoxy moiety contains one to four carbon atoms and the alkyl moiety contains one to four carbon atoms, and hydroxyalkyl of one to four carbon atoms. More preferably the 2-substituent is hydrogen, methyl, butyl, propyl hydroxymethyl, ethoxymethyl or methoxyethyl.

In instances where n can be zero, one, or two, n is preferably zero or one. The compound of the invention may be defined by Formula XII below: 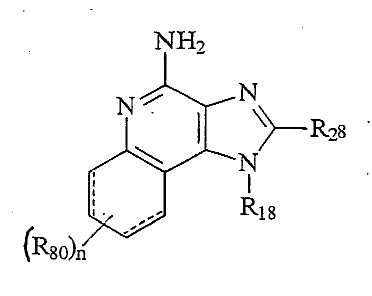 wherein
R18 is -allcyl-NR3 -SO₂ -X-R4 or -alkenyl-NR3 -SO₂ -X-R4 ;
X is a bond or -NR5 -;
R4 is aryl, heteroaryl, heterocyclyl, alkyl or alkenyl, each of which may be unsubstituted or substituted by one or more substituents selected from the group consisting of:
- alkyl;
- alkenyl;
- aryl;
- heteroaayl;
- heterocyclyl;
- substituted aryl;
- substituted heteroaryl;
- substituted heterocyclyl;
- O-alkyl;
- O-(alkyl)₀₋₁ -aryl;
- O-(alkyl)₀₋₁ -substituted aryl;
- O-(alkyl)₀₋₁ -heteroaryl;
- O-(alkyl)₀₋₁ -substituted heteroalyl;
- O-(alkyl)₀₋₁ -heterocyclyl;
- O-(alkyl)₀₋₁ -substituted heterocyclyl;
- COOH;
- CO-O-alkyl;
- CO-alkyl;
- S(O)₀₋₂ -alkyl;
- S(O)₀₋₂ -(alkyl)₀₋₁ -aryl;
- S(O)₀₋₂ -(alkyl)₀₋₁ -substituted aryl;
- S(O)₀₋₂ -(alkyl)₀₋₁ -heteroaryl;
- S(O)₀₋₂ -(alkyl)₀₋₁ -substituted heteroaryl;
- S(O)₀₋₂ -(alkyl)₀₋₁ -heterocyclyl;
- S(O)₀₋₂ -(alkyl)₀₋₁ -substituted heterocyclyl;
- (alkyl)₀₋₁ -NR3 R3;
- (alkyl)₀₋₁ -NR3 -CO-O-alkyl;
- (alkyl)₀₋₁ -NR3 -CO-alkyl;
- (alkyl)₀₋₁ -NR3 -CO-aryl;
- (aUcyl)₀₋₁ -NR3 -CO-substituted aryl;
- (alkyl)₀₋₁ -NR3 -CO-heteroaryl;
- (alkyl)₀₋₁ -NR3 -CO-substituted heteroaryl;
- N₃;
- halogen;
- haloalkyl;
- haloalkoxy;
- CO-haloalkoxy;
- NO₂;
- CN;
- OH;
- SH; and in the case of alkyl, alkenyl, or heterocyclyl, oxo;

R28 is selected from the group consisting of:
-hydrogen;
-alkyl;
-alkenyl;
-aryl;
-substituted aryl;
-heteroaryl;
-substituted heteroaryl;
-alk-yl-O-alkyl;
-allcyl-O-alkenyl; and
-alkyl or alkenyl substituted by one or more substituents selected from the group consisting of:
   - OH;
   - halogen;
   - N(R3)₂ ;
   - CO-N(R3)₂ ;
   - CO-C₁₋₁₀ alkyl;
   - CO-O-C₁₋₁₀ alkyl;
   - N₃;
   - aryl;
   - substituted aryl;
   - heteroaryl;
   - substituted heteroaryl;
   - heterocyclyl;
   - substituted heterocyclyl;
   - CO-aryl;
   - CO-(substituted aryl);
   - CO-heteroaryl; and
   - CO-(substituted heteroaryl);
   each R3 is independently selected from the group consisting of hydrogen and C₁₋₁₀ alkyl;
R5 is selected from the group consisting of hydrogen and C₁₋₁₀ alkyl, or R4 and R5 can combine to form a 3 to 7 membered heterocyclic or substituted heterocyclic ring; n is 0 to 4 and each R80 present is independently selected from the group consisting of C₁₋₁₀ alkyl, C₁₋₁₀ alkoxy, halogen and trifluoromethyl, or a pharmaceutically acceptable salt thereof.

The compounds of the present invention may be further defined by Formula (XIII) below: 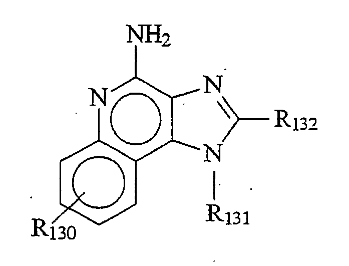
wherein:
   R₁₃₁ is selected from the group consisting of straight chain or branched chain alkyl containing one to six carbon atoms and substituted straight chain or branched chain alkyl containing one to six carbon atoms, wherein the substituent is selected from halogen, amino, mono-alkyl amino, di-alkyl amino, alkoxy, alkylthio, hydroxy or hydroxyalkyl, the alkyl groups of the substituents comprising from one to four carbon atoms;
   R₁₃₂ is selected from the group consisting of hydrogen, straight chain or branched chain alkyl containing one to six carbon atoms and substituted straight chain or branched chain alkyl containing one to six carbon atoms, wherein the substituent is X-R' wherein X is -NR"-, -O- or -S-, R' is hydrogen or straight chain or branched chain alkyl containing one to four carbon atoms and R" is hydrogen or straight chain or branched chain alkyl containing one to four carbon atoms; and
   R₁₃₀ is selected from the group consisting of hydrogen, straight chain or branched chain alkoxy containing one to four carbon atoms, halogen, and straight chain or branched chain alkyl containing one to four carbon atoms.

Preferred compounds of the invention are compounds of Formula (XIII) wherein:
R₁₃₁ is selected from the group consisting of straight chain or branched chain alkyl containing one to six carbon atoms and substituted straight chain or branched chain alkyl containing one to six carbon atoms, wherein the substituent is selected from alkoxy, hydroxy or hydroxyalkyl, the alkyl groups of the substituents comprising from one to four carbon atoms;
R₁₃₂ is selected from the group consisting of hydrogen, straight chain or branched chain alkyl containing one to six carbon atoms and substituted straight chain or branched chain alkyl containing one to six carbon atoms, wherein the substituent is X-R' wherein X is -O- and R' is hydrogen or straight chain or branched chain alkyl containing one to four carbon atoms; and
R₁₃₀ is selected from the group consisting of hydrogen, straight chain or branched chain alkoxy containing one to four carbon atoms and straight chain or branched chain alkyl containing one to four carbon atoms;

Further preferred compounds of the invention are compounds of Formula (XIII) wherein:
R₁₃₁ is selected from the group consisting of straight chain or branched chain alkyl containing one to four carbon atoms and substituted straight chain or branched chain alkyl containing one to four carbon atoms, wherein the substituent is hydroxy or hydroxyalkyl of one to four carbon atoms;
R₁₃₂ is selected from the group consisting of hydrogen, straight chain or branched chain alkyl containing one to four carbon atoms and substituted straight chain or branched chain alkyl containing one to four carbon atoms, wherein the substituent is X-R' wherein X is -O- and R' is straight or branched chain alkyl containing one or two carbon atoms; and
R₁₃₀ is hydrogen.

As used herein (and in particular with reference to Formula XII), unless defined otherwise the terms "alkyl", "alkenyl", "alkynyl" and the prefix "-alk" are inclusive of both straight chain and branched chain groups and of cyclic groups, i.e. cycloalkyl and cycloalkenyl. Unless otherwise specified, these groups contain from 1 to 20 carbon atoms, with alkenyl and alkenyl groups containing from 2 to 20 carbon atoms. Preferred groups have a total of up to 10 carbon atoms. Cyclic groups can be monocyclic or polycyclic and preferably have from 3 to 10 ring carbon atoms. Exemplary cyclic groups include cyclopropyl, cyclopentyl, cyclohexyl and adamantyl.

The term "haloalkyl" is inclusive of groups that are substituted by one or more halogen atoms, including groups wherein all of the available hydrogen atoms are replaced by halogen atoms. This is also true of groups that include the prefix "haloalk-"Examples of suitable haloalkyl groups are chloromethyl, trifluoromethyl, and the like.

The term "aryl" as used herein includes carbocyclic aromatic rings or ring systems. Examples of aryl groups include phenyl, naphthyl, biphenyl, fluorenyl and indenyl. The term "heteroaryl" includes aromatic rings or ring systems that contain at least one ring hetero atom (e.g., O, S, N). Suitable heteroaryl groups include furyl, thienyl, pyridyl, quinolinyl, tetrazolyl, imidazo, pyrazolo, thiazolo, oxazolo, and the like.

"Heterocyclyl" includes non-aromatic rings or ring systems that contain at least one ring hetero atom (e.g., O, S, N). Exemplary heterocyclic groups include pyrrolidinyl, tetrahydrofuranyl, morpholinyl, thiomorpholinyl, piperidinyl, piperazinyl, thiazolidinyl, imidazolidinyl, and the like.

Unless otherwise specified, the terms "substituted cycloalkyl", "substituted aryl", "substituted heteroaryl" and "substituted heterocyclyl" indicate that the rings or ring systems in question are further substituted by one or more substituents independently selected from the group consisting of alkyl, alkoxy, alkylthio, hydroxy, halogen, haloalkyl, haloalkylcarbonyl, haloalkoxy (e.g., trifluoromethoxy), nitro, alkylcarbonyl, alkenylcarbonyl, arylcarbonyl, heteroarylcarbonyl, aryl, arylalkyl, heteroalyl, heteroarylalkyl, heterocyclyl, heterocycloalkyl, nitrile, alkoxycarbonyl, alkanoyloxy, alkanoylthio, and in the case of cycloalkyl and heterocyclyl, oxo.

A preferred compound of the invention is imiquimod:

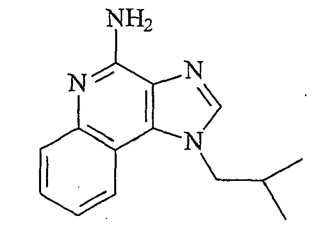

Imiquimod is 1-(2-methyl-propyl)-1H-imidazo [4,5-c] quinolin-4-amine. It has a molecular formula of C₁₄H₁₆N₄ and a molecular weight of 240.3.

A preferred compound of Formula (XIII) is resiquimod:

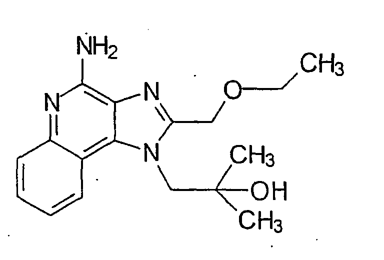

Resiquimod is 4-amino-2-ethoxymethyl-alpha, alpha-dimethyl-1H-imidazo [4,5-c] quinoline-1-ethanol. (R-848; S-28463)

The compound of the invention may be prepared by known means, for example as described in US-B1-6,245,776, US-A-4,689,338, US-A-5,389,640, US-A-5,268,376, US-A-4,929,624, US-A-5,266,575, US-A-5,352,784, US-A-5,494,916, US-A-5,482,936, US-A-5,346,905, US-A-5,395,937, US-A-5,238,944, US-A-5,525,612,US-B1-6,323,200, US-B1-6,331,539 and WO 99/29693.

The polynucleotide encoding antigen may be in the form of a vaccine composition. The term "vaccine composition" intends any pharmaceutical composition containing polynucleotide encoding an antigen, which composition can be used to prevent or treat a disease or condition in a subject.

The compound is delivered topically or transdermally. Generally, such topical delivery will be transdermal delivery. The term "transdermal" delivery intends intradermal (e. g., into the dermis or epidermis), transdermal (e. g., "percutaneous") and transmucosal administration, i.e., delivery by passage of an agent into or through skin or mucosal tissue. See, e.g., Transdermal Drug Delivery : Developmental Issues and Research Initiatives, Had graft and Guy (eds.), Marcel Dekker, Inc., (1989); Controlled Drug Delivery : Fundamentals and Applications, Robinson and Lee (eds.), Marcel Dekker Inc., (1987); and Transdermal Delivery of Drugs, Vols. 1- 3, Kydonieus and Berner (eds.), CRC Press, (1987).

Thus, topical or transdermal delivery encompasses delivery of particles from a particle delivery device (e.g. needleless syringe) as described in U. S. Patent No. 5,630,796, as well as particle-mediated delivery of coated core carriers as described in U. S. Patent No. 5,865,796.

By "core carrier" is meant a carrier particle on which a nucleic acid (e.g.,DNA) is coated in order to impart a defined particle size as well as a sufficiently high density to achieve the momentum required for cell membrane penetration, such that the nucleic acid can be delivered using particle-mediated delivery techniques, for example those described in U. S. Patent No. 5,100,792. Core carriers typically include materials such as tungsten, gold, platinum, ferrite, polystyrene and latex. See, for example, Particle Bombardment Technology for Gene Transfer, (1994) Yang, N. ed., Oxford University Press, New York, NY pages 10-11.

By "particle delivery device," or "needleless syringe," is meant an instrument which delivers a particulate composition transdermally, without a conventional needle that pierces the skin. Particle delivery devices for use with the present invention are discussed throughout this document.

By "antigen" is meant a molecule which contains one or more epitopes that will stimulate a host's immune system to make a cellular antigen-specific immune response, or a humoral antibody response. Thus, antigens include proteins, polypeptides, antigenic protein fragments, oligosaccharides, polysaccharides, and the like. Furthermore, the antigen can be derived from any known organism or pathogen, e.g. virus, bacterium, parasite, plants, protozoans, or fungus, and can be a whole organism. The term also includes tumor antigens. Similarly, an oligonucleotide or polynucleotide which expresses an antigen, such as in DNA immunization applications, is also included in the definition of antigen.

Synthetic antigens are also included, for example, polyepitopes, flanking epitopes, and other recombinant or synthetically derived antigens (Bergmann et al. (1993) Eur. J. Immunol. 23: 2777-2781; Bergmann et al. (1996) J. Immunol.157: 3242-3249 ; Suhrbier, A. (1997) Immunol. and Cell Biol. 75: 402-408; Gardner et al. (1998) 12th World AIDS Conference, Geneva, Switzerland, June 28-July 3, 1998).

The term "peptide" is used in it broadest sense to refer to a compound of two or more subunit amino acids, amino acid analogs, or other peptidomimetics. The subunits may be linked by peptide bonds or by other bonds, for example ester, ether, etc.

As used herein, the term "amino acid" refers to either natural and/or unnatural or synthetic amino acids, including glycine and both the D or L optical isomers, and amino acid analogs and peptidomimetics. A peptide of three or more amino acids is commonly called an oligopeptide if the peptide chain is short. If the peptide chain is long, the peptide is typically called a polypeptide or a protein.

The antigen typically comprises one or more T cell epitopes. "T cell epitopes" are generally those features of a peptide structure capable of inducing a T cell response. In this regard, it is accepted in the art that T cell epitopes comprise linear peptide determinants that assume extended conformations within the peptide-binding cleft of MHC molecules, (Unanue et al. (1987) Science 236: 551-557). As used herein, a T cell epitope is generally a peptide having about 8-15, preferably 5-10 or more amino acid residues.

The compound acts as adjuvant. However, further adjuvants may also be used in the method of the invention. Typically, such further adjuvants co-administered with the polynucleotide which expresses the antigen. As used herein the term "adjuvant" refers to any material that enhances the action of a drug, antigen, polynucleotide, vector or the like.

Thus, one example of an adjuvant is a "cytokine." As used herein, the term "cytokine" refers to any one of the numerous factors that exert a variety of effects on cells, for example, inducing growth, proliferation or maturation. Certain cytokines, for example TRANCE, flt-3L, and CD40L, enhance the immunostimulatory capacity of APCs. Non-limiting examples of cytokines which may be used alone or in combination include, interleukin-2 (IL-2), stem cell factor (SCF), interleukin 3 (IL-3), interleukin 6 (IL-6), interleukin 12 (IL-12), G-CSF, granulocyte macrophage-colony stimulating factor (GM-CSF), interleukin-1 alpha (IL-1 a), interleukin-11 (IL-11), MIP-1a, leukemia inhibitory factor (LIF), c-kit ligand, thrombopoietin (TPO), CD40 ligand (CD40L), tumor necrosis factor-related activation-induced cytokine (TRANCE) and flt3 ligand (flt-3L). Cytokines are commercially available from several vendors such as, for example, Genzyme (Framingham, MA), Genentech (South San Francisco, CA),Amgen (Thousand Oaks, CA), R & D Systems and Immunex (Seattle, WA).

The sequence of many of these molecules are also available, for example, from the GenBank database. It is intended, although not always explicitly stated, that molecules having similar biological activity as wild-type or purified cytokines (e. g., recombinantly produced or mutants thereof) and nucleic acid encoding these molecules are intended to be used within the spirit and scope of the invention.

A composition which contains a selected antigen and an adjuvant or a vaccine composition which is co-administered with an adjuvant, displays "enhanced immunogenicity "when it possesses a greater capacity to elicit an immune response than the immune response elicited by an equivalent amount of the antigen administered without the adjuvant. Thus, a vaccine composition may display "enhanced immunogenicity" because the antigen is more strongly immunogenic or because a lower dose or fewer doses of antigen are necessary to achieve an immune response in the subject to which the antigen is administered.

Such enhanced immunogenicity can be determined by administering the adjuvant composition and antigen controls to animals and comparing antibody titers and/or cellular-mediated immunity between the two using standard assays such as radioimmunoassay, ELISAs, CTL assays, and the like, well known in the art.

The terms "nucleic acid molecule" and "polynucleotide" are used interchangeably and refer to a polymeric form of nucleotides of any length, either deoxyribonucleotides or ribonucleotides, or analogs thereof. Non-limiting examples of polynucleotides include a gene, a gene fragment, exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, and primers.

A polynucleotide is typically composed of a specific sequence of four nucleotide bases: adenine (A); cytosine (C); guanine (G); and thymine (T) (uracil (U) is substituted for thymine (T) when the polynucleotide is RNA). Thus, the term polynucleotide sequence is the alphabetical representation of a polynucleotide molecule. This alphabetical representation can be input into databases in a computer having a central processing unit and used for bioinformatics applications such as functional genomics and homology searching.

A "gene" as used in the context of the present invention is a sequence of nucleotides in a genetic nucleic acid (chromosome, plasmid, etc.) with which a genetic function is associated. A gene is a hereditary unit, for example of an organism, comprising a polynucleotide sequence (e. g., a DNA sequence for mammals) that occupies a specific physical location (a "gene locus" or "genetic locus") within the genome of an organism. A gene can encode an expressed product, such as a polypeptide or a polynucleotide (e. g., tRNA). Alternatively, a gene may define a genomic location for a particular event/function, such as the binding of proteins and/or nucleic acids (e. g., phage attachment sites), wherein the gene does not encode an expressed product. Typically, a gene includes coding sequences, such as polypeptide encoding sequences, and non-coding sequences, such as promoter sequences, poly-adenlyation sequences, transcriptional regulatory sequences (e. g., enhancer sequences). Many eucaryotic genes have "exons" (coding sequences) interrupted by "introns" (non-coding sequences). In certain cases, a gene may share sequences with another gene (s) (e.g., overlapping genes).

A "coding sequence" or a sequence which "encodes" a selected polypeptide, is a nucleic acid molecule which is transcribed (in the case of DNA) and translated (in the case of mRNA) into a polypeptide in vivo when placed under the control of appropriate regulatory sequences (or "control elements").

The boundaries of the coding sequence are determined by a start codon at the 5' (amino) terminus and a translation stop codon at the 3' (carboxy) terminus. A coding sequence can include, but is not limited to, cDNA from viral, procaryotic or eucaryotic mRNA, genomic DNA sequences from viral or procaryotic DNA, and even synthetic DNA sequences. A transcription termination sequence may be located 3' to the coding sequence. Transcription and translation of coding sequences are typically regulated by "control elements," including, but not limited to, transcription promoters, transcription enhancer elements, transcription termination signals, polyadenylation sequences (located 3' to the translation stop codon), sequences for optimization of initiation of translation (located 5' to the coding sequence), and translation termination sequences.

A "promoter" is a nucleotide sequence which initiates transcription of a polypeptide-encoding polynucleotide. Promoters can include inducible promoters (where expression of a polynucleotide sequence operably linked to the promoter is induced by an analyte, cofactor, regulatory protein, etc.), repressible promoters (where expression of a polynucleotide sequence operably linked to the promoter is repressed by an analyte, cofactor, regulatory protein, etc.), and constitutive promoters. In addition, such promoters can also have tissue specificity.

It is intended that the term "promoter" or "control element" includes full-length promoter regions and functional (e. g., controls transcription or translation) segments of these regions. The promoter present in the polynucleotide which is capable of expressing antigen is generally capable of causing expression of the coding sequence in a cell which is found in the vicinity of the skin.

The polynucleotide capable of expressing the antigen may be delivered in the form of a vector. A "vector" is capable of transferring gene sequences to target cells (e. g., viral vectors, non-viral vectors, particulate carriers, and liposomes). Typically, "vector construct," "expression vector," and "gene transfer vector," mean any nucleic acid construct capable of directing the expression of a gene of interest and which can transfer gene sequences to target cells. Thus, the term includes cloning and expression vehicles, as well as viral vectors.

An "isolated polynucleotide" molecule is a nucleic acid molecule separate and discrete from the whole organism with which the molecule is found in nature; or a nucleic acid molecule devoid, in whole or part, of sequences normally associated with it in nature; or a sequence, as it exists in nature, but having heterologous sequences (as defined below) in association therewith.

"Operably linked" refers to an arrangement of elements wherein the components so described are configured so as to perform their usual function. Thus, a given promoter that is operably linked to a coding sequence (e. g., an antigen or interest) is capable of effecting the expression of the coding sequence when the regulatory proteins and proper enzymes are present. In some instances, certain control elements need not be contiguous with the coding sequence, so long as they function to direct the expression thereof. For example, intervening untranslated yet transcribed sequences can be present between the promoter sequence and the coding sequence and the promoter sequence can still be considered "operably linked" to the coding sequence.

"Recombinant" as used herein to describe a nucleic acid molecule means a polynucleotide of genomic, cDNA, semisynthetic, or synthetic origin which, by virtue of its origin or manipulation: (1) is not associated with all or a portion of the polynucleotide with which it is associated in nature; and/or (2) is linked to a polynucleotide other than that to which it is linked in nature. The term "recombinant" as used with respect to a protein or polypeptide means a polypeptide produced by expression of a recombinant polynucleotide.

Protein antigens may have homology and/or sequence identity with naturally occurring antigens, such as any of the antigens mentioned herein. Similarly polynucleotides capable of expressing antigen will have sequence which has homology and/or sequence identity with naturally occurring sequences, such as those which encode the antigens mentioned herein. Techniques for determining nucleic acid and amino acid "sequence identity" also are known in the art. Typically, such techniques include determining the nucleotide sequence of the mRNA for a gene and/or determining the amino acid sequence encoded thereby, and comparing these sequences to a second nucleotide or amino acid sequence.

In general, "identity" refers to an exact nucleotide-to-nucleotide or amino acid-to-amino acid correspondence of two polynucleotides or polypeptide sequences, respectively. Two or more sequences (polynucleotide or amino acid) can be compared by determining their "percent identity." The percent identity of two sequences, whether nucleic acid or amino acid sequences, is the number of exact matches between two aligned sequences divided by the length of the shorter sequences and multiplied by 100.

An approximate alignment for nucleic acid sequences is provided by the local homology algorithm of Smith and Waterman, Advances in Applied Mathematics 2: 482-489 (1981). This algorithm can be applied to amino acid sequences by using the scoring matrix developed by Dayhoff, Atlas of Protein Sequences and Structure, M. O. Dayhoff ed., 5 suppl. 3: 353-358, National Biomedical Research Foundation, Washington, D. C., USA, and normalized by Gribskov, Nucl. AcidsRes. 14 (6): 6745-6763 (1986). An exemplary implementation of this algorithm to determine percent identity of a sequence is provided by the Genetics Computer Group (Madison, WI) in the "BestFit" utility application. The default parameters for this method are described in the Wisconsin Sequence Analysis PackageProgram Manual, Version 8 (1995) (available from Genetics Computer Group, Madison, WI). A preferred method of establishing percent identity in the context of the present invention is to use the MPSRCH package of programs copyrighted by the University of Edinburgh, developed by John F. Collins and Shane S.

Sturrok, and distributed by IntelliGenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match"value reflects"sequence identity. "Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff= 60; expect = 10; Matrix = BLOSUM62 ; Descriptions = 50 sequences; sort by = HIGH SCORE; Databases = non-redundant, GenBank +EMBL + DDBJ + PDB + GenBank CDS translations + Swiss protein + Spupdate + PIR. Details of these programs can be found at the following internet address: http://www. ncbi. nlm. gov/cgi-bin/BLAST.

Alternatively, homology can be determined by hybridization of polynucleotides under conditions which form stable duplexes between homologous regions, followed by digestion with single-stranded-specific nuclease (s), and size determination of the digested fragments. Two DNA, or two polypeptide sequences are" substantially homologous"to each other when the sequences exhibit at least about 80%-85%, preferably at least about 90%, and most preferably at least about 95%-98% sequence identity over a defined length of the molecules, as determined using the methods above.

As used herein, substantially homologous also refers to sequences showing complete identity to the specified DNA or polypeptide sequence. DNA sequences that are substantially homologous can be identified in a Southern hybridization experiment under, for example, stringent conditions, as defined for that particular system. For example, stringent hybridization conditions can include 50% formamide, 5x Denhardt's Solution, 5x SSC, 0.1% SDS and 100 pg/ml denatured salmon sperm DNA and the washing conditions can include 2x SSC, 0.1% SDS at 37 C followed by 1x SSC, 0.1 % SDS at 68 C. Defining appropriate hybridization conditions is within the skill of the art. See, e. g., Sambrook et al., supra ; DNA Cloning, supra ; Nucleic Acid Hybridization, supra.

As used herein, the term "treatment" includes any of following: the prevention of infection or reinfection; the reduction or elimination of symptoms; and the reduction or complete elimination of a pathogen. Treatment may be effected prophylactically (prior to infection) or therapeutically (following infection). An "effective amount" is an amount sufficient to effect beneficial or desired results. An effective amount can be administered in one or more administrations, applications of dosages. The term "co-administering" or "coadministration" refers to administration of at least two substances. Coadministration can be achieved by administering the substances concurrently or at different times. In addition, co-administration includes delivery using one or more delivery means.

The invention is carried out for the purpose of stimulating a suitable immune response. By suitable immune response, it is meant that the invention can bring about in an immunized subject an immune response characterized by the production of T lymphocytes specific for an antigen, wherein the immune response can protect the subject against subsequent infection with homologous or heterologous strains of the relevant pathogen, reduce pathogen burden, bring about resolution of infection in a shorter amount of time relative to a non-immunized subject, or prevent or reduce clinical manifestation of disease symptoms.

The subject on which the invention is performed is generally a vertebrate subject. By "vertebrate subject" is meant any member of the subphylum cordata, particularly mammals, including, without limitation, humans and other primates, as well as rodents, such as mice and rats. The term does not denote a particular age. Thus, both adult and newborn individuals are intended to be covered. In one embodiment the subject is susceptible to or at risk from the relevant disease.

A polynucleotide which is capable of expressing antigen is also termed a DNA-vaccine (although as is clear from the disclosure herein this may consist of a polynucleotide other than DNA). DNA-vaccines generally consist of a plasmid that encodes a relevant antigen for de novo synthesis by cells present in a targeted tissue. Viral promoters, e. g., the promoter from Cytomegalovirus (CMV), are generally used in DNA-vaccine plasmid constructs to drive antigen expression. Delivery of these DNA-vaccine plasmids, both in "naked" form and attached to particles, has been shown to elicit both humoral and cell-mediated immune responses. (See, e. g., Wang et al. (1993) Proc. Natl. Acad. Sci. USA 90 : 4156-4160; Tang et al. (1992) Nature 356: 152-154; Fynan, supra).

The polynucleotides may be introduced into cells in vivo by, for example, transfection or by coating the polynucleotides onto particles and administering the coated particles to the cells. Alternatively, the polynucleotides may be provided in a particulate (e. g., powder) form, discussed more fully below and in the disclosure of International Publication Numbers WO 97/48485 and WO 98/10750.

Thus, the invention elicits an immune response, including a CTL response (typically CD8 T cell response), in a vertebrate subject by administering a selected antigen or a polynucleotide encoding a selected antigen, where the antigen encoding sequence is operably linked to a regulatory element capable of causing expression of the coding sequence.

### Antigens

The methods described herein elicit an immune response against a wide variety of antigens for the treatment and/or prevention of a number of conditions including, but not limited to, cancer, allergies, toxicity and infection by pathogens such as viruses, bacteria, fungi, and other pathogenic organisms.

Suitable viral antigens for use in the present invention include, but are not limited to, those obtained or derived from the hepatitis family of viruses, including hepatitis A virus (HAV), hepatitis B virus (HBV), hepatitis C virus (HCV), the delta hepatitis virus (HDV), hepatitis E virus (HEV) and hepatitis G virus (HGV). See, e. g., International Publication Nos. WO 89/04669;WO 90/11089; and WO 90/14436. The HCV genome encodes several viral proteins, including E1 and E2. See, e. g., Houghton et al. (1991) Hepatology 14 : 381-388 Nucleic acid molecules containing sequences encoding these proteins, as well as antigenic fragments thereof, will find use in the present methods. Similarly, the coding sequence for the 8-antigen from HDV is known (see, e. g., U. S. Patent No. 5,378,814).

In like manner, a wide variety of proteins from the herpesvirus family can be used as antigens in the present invention, including proteins derived from herpes simplex virus (HSV) types 1 and 2, such as HSV-1 and HSV-2 glycoproteins gB, gD and gH; antigens from varicella zoster virus (VZV), Epstein-Barr virus (EBV) and cytomegalovirus (CMV) including CMV gB and gH; and antigens from other human herpesviruses such as HHV6 and HHV7. (See, e. g. Chee et al. (1990) Cytomegaloviruses (J. K. McDougall, ed., Springer Verlag, pp. 125-169; McGeoch et al. (1988) J. Gen. Virol. 69: 1531-1574; U. S. Patent No. 5,171,568; Baer et al. (1984) Nature 310: 207-211; and Davison et al. (1986) J. Gen. Viol. 67: 1759-1816.) Human immunodeficiency virus (HIV) antigens, such as gp120 molecules for a multitude of HIV-1 and HIV-2 isolates, including members of the various genetic subtypes of HIV, are known and reported (see, e. g., Myers et al., Los Alamos Database, Los Alamos National Laboratory, Los Alamos, New Mexico (1992); and Modrow et al. (1987) J. Virol. 61: 570-578) and antigen-containing nucleic acid sequences derived or obtained from any of these isolates will find use in the present invention.

Furthermore, other immunogenic proteins derived or obtained from any of the various HIV isolates will find use herein, including sequences encoding one or more of the various envelope proteins such as gap 160 and gp41, gag antigens such as p24gag and p55gag, as well as proteins derived from the pol, env, tat, vif, rev, nef, vpr, vpu and LTR regions of HIV.

Antigens derived or obtained from other viruses will also find use herein, such as without limitation, antigens from members of the families Picornaviridae (e. g., polioviruses, rhinoviruses, etc.); Caliciviridae; Togaviridae (e. g., rubella virus, dengue virus, etc.); Flaviviridae; Coronaviridae; Reoviridae (e. g., rotavirus, etc.); Bimaviridae ; Rhabodoviridae (e. g., rabies virus, etc.); Orthomyxoviridae (e. g., influenza virus types A, B and C, etc.); Filoviridae; Paramyxoviridae (e.g., mumps virus, measles virus, respiratory syncytial virus, parainfluenza virus, etc.); Bunyaviridae; Arenaviridae; Retroviradae (e. g., HTLV-I ; HTLV-11 ; HIV-1 (also known as HTLV-111, LAV, ARV, hTLR, etc.)), including but not limited to antigens from the isolates HIVIllb, HIVSF2, HTVLAV, HIVLAI, HIVMN); HIV-1CM235, HIV-1 ; HIV-2, among others; simian immunodeficiency virus (SIV); Papillomavirus, the tick-bourne encephalitis viruses; and the like. See, e. g.Virology, 3rd Edition (W. K. Joklik ed. 1988); Fundamental Virology, 2nd Edition (B. N. Fields and D. M. Knipe, eds. 1991), for a description of these and other viruses.

In some contexts, it may be preferable that a selected antigen is obtained or derived from a viral pathogen that typically enters the body via a mucosal surface and is known to cause or is associated with human disease, such as, but not limited to, HIV (AIDS), influenza viruses (Flu), herpes simplex viruses (genital infection, cold sores, STDs), rotaviruses (diarrhea), parainfluenza viruses (respiratory infections), poliovirus (poliomyelitis), respiratory syncytial virus (respiratory infections), measles and mumps viruses (measles, mumps), rubella virus (rubella), and rhinoviruses (common cold).

Suitable bacterial and parasitic antigens can be obtained or derived from known causative agents responsible for diseases including, but not limited to, Diptheria, Pertussis, Tetanus, Tuberculosis, Bacterial or Fungal Pneumonia, Otitis Media, Gonnorhea, Cholera, Typhoid, Meningitis, Mononucleosis, Plague, Shigellosis or Salmonellosis, Legionaire's Disease, Lyme Disease, Leprosy, Malaria, Hookworm, Onchocerciasis, Schistosomiasis, Trypamasomialsis, Lesmaniasis, Giardia, Amoebiasis, Filariasis, Borelia, and Trichinosis. Still further antigens can be obtained or derived from unconventional pathogens such as the causative agents of kuru, Creutzfeldt-Jakob disease (CJD), scrapie, transmissible mink encephalopathy, and chronic wasting diseases, or from proteinaceous infectious particles such as prions that are associated with mad cow disease.

Specific pathogens from which antigens can be derived include M. tuberculosis, Chlamydia, N. gonorrhoeae, Shigella, Salmonella, Vibrio Cholera, Treponema pallidua, Pseudomonas, Bordetella pertussis, Brucella, Franciscella tulorensis, Helicobacter pylori, Leptospria interrogaus, Legionella pneumophila, Yersinia pestis, Streptococcus (types A and B), Pneumococcus, Meningococcus, Hemophilus influenza (type b), Toxoplasma gondic, Complylobacteriosis, Moraxella catarrthalis, Donovanosis, and Actinomycosis ; fungal pathogens including Candidiasis and Aspergillosis; parasitic pathogens including Taenia, Flukes, Roundworms, Amebiasis, Giardiasis, Cryptosporidium, Schistosoma, Pneumocystis carinii, Trichomoniasis and Trichinosis. Thus, the present invention can also be used to provide a suitable immune response against numerous veterinary diseases, such as Foot and Mouth diseases, Coronavirus, Pasteurella multocida, Helicobacter, Strongylus vulgaris, Actinobacillus pleuropneumonia, Bovine viral diarrhea virus (BVDV), Klebsiella pneumoniae, E. coli, Bordetella pertussis, Bordetella parapertussis and brochiseptica.

Typically, a nucleotide sequence corresponding to (encoding) one or more of the above-listed antigen (s) is used in the production of the polynucleotides, as described below.

### Isolation of Genes and Construction of Polynucleotides

The polynucleotides of the invention encode at least one antigen (e.g., antigens derived from and/or expressed by viruses, bacteria, fungi, worms, toxins, allergens or cancer cells) operably linked to a promoter, such as a viral, non-viral, cell-or tissue-specific promoter (e. g., a promoter derived from a regulatory element which controls transcription of a sequence in cells of the species of subject to be vaccinated).

These polynucleotides are useful in eliciting an immune response to the antigen (s), particularly in activating T-lymphocytes. Nucleotide sequences selected for use in the present invention can be derived from known sources, for example, by isolating the same from cells containing a desired gene or nucleotide sequence using standard techniques.

Similarly, the nucleotide sequences can be generated synthetically using standard modes of polynucleotide synthesis that are well known in the art. See, e. g., Edge et al. (1981) Nature 292: 756-762; Nambair et al. (1994) Science 223: 1299-1301 ; Jay et al. (1984) J. Biol. Chem. 259 : 6311-6317. Generally, synthetic oligonucleotides can be prepared by either the phosphotriester method as described by Edge et al., supra, and Duckworth et al. (1981) Nucleic Acids Res. 9 : 1691-1706, or the phosphoramidite method as described by Beaucage et al.(1981) Tet. Letts. 22: 1859, and Matteucci et al. (1981) J. Am. Chem. Soc. 103: 3185. Synthetic oligonucleotides can also be prepared using commercially available automated oligonucleotide synthesizers. The nucleotide sequences can thus be designed with appropriate codons for a particular amino acid sequence.

In general, one will select preferred codons for expression in the intended host. The complete sequence is assembled from overlapping oligonucleotides prepared by standard methods and assembled into a complete coding sequence. See, e. g., Edge et al. (supra) ; Nambair et al. (supra) and Jay et al. (supra). Another method for obtaining nucleic acid sequences for use herein is by recombinant means. Thus, a desired nucleotide sequence can be excised from a plasmid carrying the same using standard restriction enzymes and procedures.

Site specific DNA cleavage is performed by treating with the suitable restriction enzyme (or enzymes) under conditions which are generally understood in the art, and the particulars of which are specified by manufacturers of commercially available restriction enzymes. If desired, size separation of the cleaved fragments may be performed by polyacrylamide gel or agarose gel electrophoresis using standard techniques.

Restriction cleaved fragments may be blunt ended by treating with the large fragment of E. coli DNA polymerase I (Klenow) in the presence of the four deoxynucleotide triphosphates (dNTPs) using standard techniques. The Klenow fragment fills in at 5'single-stranded overhangs but digests protruding 3'single strands, even though the four dNTPs are present. If desired, selective repair can be performed by supplying only one, or several, selected dNTPs within the limitations dictated by the nature of the overhang. After Klenow treatment, the mixture can be extracted with e. g. phenol/chloroform, and ethanol precipitated.

Treatment under appropriate conditions with S 1 nuclease or BAL-31 results in hydrolysis of any single-stranded portion.

Yet another convenient method for isolating specific nucleic acid molecules is by the polymerase chain reaction (PCR). Mullis et al. (1987) Methods Enzymol. 155: 335-350. This technique uses DNA polymerase, usually a thermostable DNA polymerase, to replicate a desired region of DNA. The region of DNA to be replicated is identified by oligonucleotides of specified sequence complementary to opposite ends and opposite strands of the desired DNA to prime the replication reaction. The product of the first round of replication is itself a template for subsequent replication, thus repeated successive cycles of replication result in geometric amplification of the DNA fragment delimited by the primer pair used. This method also allows for the facile addition of nucleotide sequences onto the ends of the DNA product by incorporating these added sequences onto the oligonucleotide primers (see, e. g., PCR Protocols, A Guide to Methods and Applications, Innis et al (eds) Harcourt Brace Jovanovich Publishers, NY (1994)). PCR conditions used for each amplification reaction are empirically determined. A number of parameters influence the success of a reaction. Among them are annealing temperature and time, extension time, Mg2+ and ATP concentration, pH, and the relative concentration of primers, templates, and deoxyribonucleotides.

Once coding sequences for desired proteins have been prepared or isolated, such sequences can be cloned into any suitable vector or replicon. Numerous cloning vectors are known to those of skill in the art, and the selection of an appropriate cloning vector is a matter of choice. Ligations to other sequences are performed using standard procedures, known in the art.

As described in detail below, selected nucleotide sequences can be placed under the control of regulatory sequences such as a promoter, so that the sequence encoding the desired protein is transcribed into RNA in the host tissue transformed by a vector containing this expression construct.

### Promoters

Expression of a selected antigen in a polynucleotide used in the invention is driven by a promoter, such as a viral, non-viral, preferably mammalian, cell- (or tissue-) specific promoter. In a preferred embodiment, in addition to promoters, it may be desirable to add other regulatory sequences which allow for regulation of the expression of protein sequences encoded by the delivered nucleotide sequences. Suitable additional regulatory sequences are known to those of skill in the art, and examples include those which cause the expression of a coding sequence to be turned on or off in response to a chemical or physical stimulus, including the presence of a regulatory compound. Other types of regulatory elements may also be present in the vector, for example, enhancer sequences.

An expression vector is constructed so that the particular coding sequence is located in the vector with the appropriate regulatory sequences such that the positioning and orientation of the coding sequence with respect to the control sequences allows the coding sequence to be transcribed under the" control "of the control sequences (i. e., RNA polymerase, which binds to the DNA molecule at the control sequences, transcribes the coding sequence). Modification of the sequences encoding the particular protein of interest may be desirable to achieve this end. For example, in some cases it may be necessary to modify the sequence so that it is attached to the control sequences with the appropriate orientation; i. e., to maintain the reading frame. The control sequences and other regulatory sequences may be ligated to the coding sequence prior to insertion into a vector.

Alternatively, the coding sequence can be cloned directly into an expression vector which already contains the control sequences and an appropriate restriction site.

Generally, nucleic acid molecules used in the subject methods contain coding regions with suitable control sequences and, optionally, ancillary nucleotide sequences which encode cytokines or other immune enhancing polypeptides. The nucleic acid molecules are generally prepared in the form of vectors which include the necessary elements to direct transcription and translation in a recipient cell.

### Adjuvants

In order to augment an immune response in a subject, the compositions and methods described herein can further include ancillary substances/adjuvants as well as the compound of the invention, such as pharmacological agents, cytokines, or the like. Suitable adjuvants include any substance that enhances the immune response of the subject to the antigen (including when delivered by the polynucleotide of the invention). They may enhance the immune response by affecting any number of pathways, for example, by stabilizing the antigen/MHC complex, by causing more antigen/MHC complex to be present on the cell surface, by enhancing maturation of APCs, or by prolonging the life of APCs (e. g., inhibiting apoptosis). As described herein, these cytokines, delivered as either peptides or as polynucleotides encoding functional peptides, are also be useful in eliciting immune responses.

Ancillary nucleic acid sequences coding for peptides known to stimulate, modify, or modulate a host's immune response (e. g., cytokines), can be co-administered as polynucleotides with the above-described antigen-encoding polynucleotides or peptide antigens. These nucleotides can be administered either on the same vector that carries the antigen-encoding sequence, or, alternatively on a separate vector. In some cases, it may be desirable to design a polynucleotide in which both the antigen-encoding sequence and the adjuvant-encoding sequence are under the control of the same promoter.

### Administration of Polynucleotides and Adjuvants

The polynucleotides, adjuvants and ancillary substances (including the compound of the invention) described herein may be administered by any suitable method (although the compound of the invention will be administered topically or transdermally). In a preferred embodiment, described below, they are administered by coating them onto particles and then administering the particles to the subject or cells. However, they may also be delivered using a viral vector as known in the art, or by using non-viral systems, as described for example in U. S. Patent No. 5,589,466.

### Viral Vectors

A number of viral based systems have been used for gene delivery. For example, retroviral systems are known and generally employ packaging lines which have an integrated defective provirus (the"helper") that expresses all of the genes of the virus, known as the long terminal repeats (LTRs). The gene of interest can be inserted in the vector and packaged in the viral shells synthesized by the retroviral helper. The recombinant virus can then be isolated and delivered to a subject. (See, e. g., U. S. Patent No. 5,219,740.) Representative retroviral vectors include but are not limited to vectors such as the LHL, N2, LNSAL, LSHL and LHL2 vectors described in e. g., U. S. Patent No. 5,219,740 as well as derivatives of these vectors, such as the modified N2 vector described herein. Retroviral vectors can be constructed using techniques well known in the art. See, e. g., U. S. Patent No 5,219,740; Mann et al. (1983) Cell 33 : 153-159.

Adenovirus based systems have been developed for gene delivery and are suitable for delivering the polynucleotides described herein. Human adenoviruses are double-stranded DNA viruses which enter cells by receptor mediated endocytosis. These viruses are particularly well suited for gene transfer because they are easy to grow and manipulate and they exhibit a broad host range in vivo and in vitro. For example, adenoviruses can infect human cells of hematopoietic, lymphoid and myeloid origin. Furthermore, adenoviruses infect quiescent as well as replicating target cells. Unlike retroviruses which integrate into the host genome, adenoviruses persist extrachromosomally thus minimizing the risks associated with insertional mutagenesis. The virus is easily produced at high titers and is stable so that it can be purified and stored. Even in the replication-competent form, adenoviruses cause only low level morbidity and are not associated with human malignancies. Accordingly, adenovirus vectors have been developed which make use of these advantages. For a description of adenovirus vectors and their uses see, e. g., Haj-Ahmad and Graham (1986) J. Virol. 57 : 267-274; Bett et al. (1993) J. Virol. 67: 5911-5921; Mittereder et al. (1994) Human Gene Therapy 5: 717-729; Seth et al. (1994) J. Virol. 68: 933-940;Barr et al. (1994) Gene Therapy 1: 51-58 ; Berkner, K. L. (1988) BioTechniques 6 : 616-629; Rich et al. (1993) Human Gene Therapy 4 : 461-476.

Adeno-associated viral vector (AAV) can also be used to administer the polynucleotides described herein. AAV vectors can be derived from any AAV serotype, including without limitation, AAV-1, AAV-2, AAV-3, AAV-4, AAV5, AAVX7, etc. AAV vectors can have one or more of the AAV wild-type genes deleted in whole or part, preferably the rep and/or cap genes, but retain one or more functional flanking inverted terminal repeat (ITR) sequences. Functional ITR sequences are necessary for the rescue, replication and packaging of the AAV virion. Thus, an AAV vector includes at least those sequences required in cis for replication and packaging (e. g., functional ITRs) of the virus. The ITR sequence need not be the wild-type nucleotide sequence, and may be altered, e. g., by the insertion, deletion or substitution of nucleotides, so long as the sequence provides for functional rescue, replication and packaging.

AAV expression vectors are constructed using known techniques to at least provide as operatively linked components in the direction of transcription, control elements including a transcriptional initiation region, the DNA of interest and a transcriptional termination region. The control elements are selected to be functional in a mammalian cell. The resulting construct which contains the operatively linked components is bounded (5'and 3') with functional AAV ITR sequences. Suitable AAV constructs can be designed using techniques well known in the art. See, e. g., U. S. Patent Nos. 5,173,414 and 5,139,941; International Publication Nos. WO 92/01070 (published 23 January 1992) and WO 93/03769 (published 4 March 1993); Lebkowski et al. (1988) Molec. Cell. Biol. 8: 3988-3996; Vincent et al. (1990) Vaccines 90 (Cold Spring Harbor Laboratory Press); Carter, B. J. (1992) Current Opinion in Biòòtechnology 3 : 533539; Muzyczka, N. (1992) Current Topics in Microbiol. and Immunol. 158 : 97- 129; Kotin, R. M. (1994) Human Gene Therapy 5: 793-801; Shelling and Smith (1994) Gene Therapy 1: 165-169; and Zhou et al. (1994) J. Exp. Med. 179: 18671875.

### Pharmaceutical Preparations

Formulation of a preparation comprising the polynucleotides, with or without addition of an adjuvant composition, or formulation of the compound can be carried out using standard pharmaceutical formulation chemistries and methodologies all of which are readily available to the ordinarily skilled artisan. Where appropriate, the compound may also be formulated and administered as described below.

For example, compositions containing one or more nucleic acid molecules (e. g., present in a plasmid or viral vector) can be combined with one or more pharmaceutically acceptable excipients or vehicles to provide a liquid preparation.

Auxiliary substances, such as wetting or emulsifying agents, pH buffering substances and the like, may be present in the excipient or vehicle. These excipients, vehicles and auxiliary substances are generally pharmaceutical agents that do not induce an immune response in the individual receiving the composition, and which may be administered without undue toxicity.

Pharmaceutically acceptable excipients include, but are not limited to, liquids such as water, saline, polyethyleneglycol, hyaluronic acid, glycerol and ethanol.

Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. It is also preferred, although not required, that the preparation will contain a pharmaceutically acceptable excipient that serves as a stabilizer, particularly for peptide, protein or other like molecules if they are to be included in the vaccine composition. Examples of suitable carriers that also act as stabilizers for peptides include, without limitation, pharmaceutical grades of dextrose, sucrose, lactose, trehalose, mannitol, sorbitol, inositol, dextran, and the like.

Other suitable carriers include, again without limitation, starch, cellulose, sodium or calcium phosphates, citric acid, tartaric acid, glycine, high molecular weight polyethylene glycols (PEGs), and combination thereof. A thorough discussion of pharmaceutically acceptable excipients, vehicles and auxiliary substances is available in REMINGTONS PHARMACEUTICAL SCIENCES (Mack Pub. Co., N. J. 1991).

Certain facilitators of nucleic acid uptake and/or expression ("transfection facilitating agents") can also be included in, e. g., non-viral vector compositions, for example, facilitators such as bupivacaine, cardiotoxin and sucrose, and transfection facilitating vehicles such as liposomal or lipid preparations that are routinely used to deliver nucleic acid molecules. Anionic and neutral liposomes are widely available and well known for delivering nucleic acid molecules (see, e. g., Liposomes : A Practical Approach, (1990) RPC New Ed., IRL Press).

Cationic lipid preparations are also well known vehicles for use in delivery of nucleic acid molecules. Suitable lipid preparations include DOTMA (N- [1- (2, 3dioleyloxy) propyl]-N, N, N-trimethylammonium chloride), available under the tradename Lipofectin, and DOTAP (1, 2-bis (oleyloxy)-3 (trimethylammonio) propane), see, e. g., Felgner et al. (1987) Proc. Natl. Acad. Sci. USA 84: 7413-7416; Malone et al. (1989) Proc. Natl. Acad. Sci. USA 86: 6077-6081; US Patent Nos 5,283,185 and 5,527,928, and International Publication Nos WO 90/11092, WO 91/15501 and WO 95/26356. These cationic lipids may preferably be used in association with a neutral lipid, for example DOPE (dioleyl phosphatidylethanolamine). Still further transfection-facilitating compositions that can be added to the above lipid or liposome preparations include spermine derivatives (see, e. g., International Publication No. WO 93/18759) and membrane-permeabilizing compounds such as GALA, Gramicidine S and cationic bile salts (see, e. g., International Publication No. WO 93/19768).

Alternatively, the nucleic acid molecules may be encapsulated, adsorbed to, or associated with, particulate carriers. Suitable particulate carriers include those derived from polymethyl methacrylate polymers, as well as PLG microparticles derived from poly (lactides) and poly (lactide-co-glycolides). See, e. g., Jeffery et al. (1993) Pharm. Res. 10: 362368. Other particulate systems and polymers can also be used, for example, polymers such as polylysine, polyarginine, polyomithine, spermine, spermidine, as well as conjugates of these molecules.

The formulated vaccine compositions will thus typically include polynucleotide (e. g., a plasmid) containing a sequence encoding an antigen of interest in an amount sufficient to mount an immunological response. An appropriate effective amount can be readily determined by one of skill in the art. Such an amount will fall in a relatively broad range that can be determined through routine trials. In the case of antigens 10 ug to 1 g may be used, for example 100ug to 0.1 g. Immune responses have been obtained using as little as 1 pg of DNA, while in other administrations, up to 2 mg of DNA has been used. It is generally expected that an effective dose of polynucleotides containing the genomic fragments will fall within a range of about 10 pg to 1000 ug. However, doses above and below this range may also be found effective. The compositions may thus contain from about 0.1 % to about 99.9% of the polynucleotide or compound. In the case of the compound, 1 ug to 10g is typically administered, preferably 0.1mg to 50mg, and most preferably 1mg to 5mg.

### Administration of Pharmaceutical Preparations

Typically, the compound (generally in the amounts mentioned above) is applied (topically or transdermally) to an area having a diameter of 1 to 6cm, preferably 2 to 4 cm.

The compound is generally applied at the site where the polynucleotide is administered (or close to there, such as within 2 cm of that site). In another embodiment the compound is applied to a site which is immunologically related to the site where the polynucleotide is administered, such as at a draining lymph node from the site where the polynucleotide is administered.

Administration of the above-described pharmaceutical preparations (containing the polynucleotide) can be effected in one or more doses (typically 2, 3, 4 or more doses). The compound will be administered 12 to 36 hours after at least one of the administrations of polynucleotide, for example after at least each of 2, 3, 4 or more administrations of polynucleotide.

Delivery may be via conventional needle and syringe for the liquid compositions and for liquid suspensions containing particulate compositions. In addition, various liquid jet injectors are known in the art and may be employed to administer the present compositions. Methods of determining the most effective means and dosages of administration are well known to those of skill in the art and will vary with the delivery vehicle, the composition of the therapy, the target cells, and the subject being treated. Single and multiple administrations can be carried out with the dose level and pattern being selected by the attending physician. It should be understood that more than one antigen sequence can be carried by a polynucleotide vector construct.

Alternatively, separate vectors (e. g., plasmid or viral vectors), each containing sequences expressing one or more antigens can also be delivered to a subject as described herein.

Furthermore, the polynucleotides may be combined with other suitable compositions and therapies. For instance, in order to augment an immune response in a subject, the compositions and methods described herein can further include ancillary substances (e. g., adjuvants), such as pharmacological agents, cytokines, or the like. Ancillary substances may be administered, for example, as proteins or other macromolecules at the same time, prior to, or subsequent to, administration of the polynucleotides described herein. The nucleic acid molecule compositions may also be administered directly to the subject or, alternatively, delivered ex vivo, to cells derived from the subject, using methods known to those skilled in the art.

### Coated Particles

In one embodiment, the polynucleotides (e. g., DNA vaccines), adjuvants, and/or compound are delivered using carrier particles (e.g., core carriers). Particle-mediated methods for delivering such preparations are known in the art. Thus, once prepared and suitably purified, the above-described substances can be coated onto carrier particles (e. g., core carriers) using a variety of techniques known in the art. Carrier particles are selected from materials which have a suitable density in the range of particle sizes typically used for intracellular delivery from an appropriate particle mediated delivery device. The optimum carrier particle size will, of course, depend on the diameter of the target cells. Alternatively, colloidal gold particles can be used wherein the coated colloidal gold is administered (e. g., injected) into tissue (e. g., skin or muscle) and subsequently taken-up by immune-competent cells.

For the purposes of the invention, tungsten, gold, platinum and iridium carrier particles can be used. Tungsten and gold particles are preferred. Tungsten particles are readily available in average sizes of 0.5 to 2.0, um in diameter. Although such particles have optimal density for use in particle acceleration delivery methods, and allow highly efficient coating with DNA, tungsten may potentially be toxic to certain cell types. Gold particles or microcrystalline gold (e. g., gold powder A1570, available from Engelhard Corp., East Newark, NJ) will also find use with the present methods. Gold particles provide uniformity in size (available from Alpha Chemicals in particle sizes of 1-3, um, or available from Degussa, South Plainfield, NJ in a range of particle sizes including 0.95, um) and reduced toxicity.

A number of methods are known and have been described for coating or precipitating DNA or RNA onto gold or tungsten particles. Most such methods generally combine a predetermined amount of gold or tungsten with plasmid DNA, CaCl2 and spermidine. The resulting solution is vortexed continually during the coating procedure to ensure uniformity of the reaction mixture. After precipitation of the nucleic acid, the coated particles can be transferred to suitable membranes and allowed to dry prior to use, coated onto surfaces of a sample module or cassette, or loaded into a delivery cassette for use in a suitable particle delivery instrument.

Peptide adjuvants (e. g., cytokines) and antigen, can also be coated onto suitable carrier particles, e. g., gold or tungsten. For example, peptides can be attached to the carrier particle by simply mixing the two components in an empirically determined ratio, by ammonium sulfate precipitation or other solvent precipitation methods familiar to those skilled in the art, or by chemical coupling of the peptide to the carrier particle. The coupling of L-cysteine residues to gold has been previously described (Brown et al., Chemical Society Reviews 9 : 271- 311 (1980)). Other methods include, for example, dissolving the peptide antigen in absolute ethanol, water, or an alcohol/water mixture, adding the solution to a quantity of carrier particles, and then drying the mixture under a stream of air or nitrogen gas while vortexing. Alternatively, the peptide antigens can be dried onto carrier particles by centrifugation under vacuum. Once dried, the coated particles can be resuspended in a suitable solvent (e. g., ethyl acetate or acetone), and triturated (e. g., by sonication) to provide a substantially uniform suspension.

### Administration of Coated Particles

Following their formation, carrier particles coated with either nucleic acid preparations, or peptide or protein adjuvant preparations, or the compound of the invention are delivered to a subject, for example transdermally, using particle-mediated delivery techniques. Various particle delivery devices suitable for particle-mediated delivery techniques are known in the art, and are all suited for use in the practice of the invention. Current device designs employ an explosive, electric or gaseous discharge to propel the coated core carrier particles toward target cells. The coated particles can themselves be releasably attached to a movable carrier sheet, or removably attached to a surface along which a gas stream passes, lifting the particles from the surface and accelerating them toward the target. An example of a gaseous discharge device is described in U. S. Patent No. 5,204,253. An explosive-type device is described in U. S. Patent No. 4,945,050. One example of an electric discharge-type particle acceleration apparatus is described in U. S. Patent No. 5,120,657. Another electric discharge apparatus suitable for use herein is described in U. S. Patent No. 5,149,655.

The coated particles are administered to the subject to be treated in a manner compatible with the dosage formulation, and in an amount that will be effective to bring about a desired immune response. The amount of the composition to be delivered which, in the case of nucleic acid molecules is generally in the range of from 0.001 to 100.0 ug, more typically 0.01 to 10.0, ug of nucleic acid molecule per dose, and in the case of antigen peptide or protein molecules or compound of the invention is 1, ug to 5 mg, more typically 1 to 50, ug of peptide, depends on the subject to be treated. The exact amount necessary will vary depending on the age and general condition of the individual being immunized and the particular nucleotide sequence or peptide selected, as well as other factors. An appropriate effective amount can be readily determined by one of skill in the art upon reading the instant specification.

Thus, an effective amount of the nucleic acids or compound will be sufficient to bring about a suitable immune response in an immunized subject, and will fall in a relatively broad range that can be determined through routine trials. Preferably, the coated particles are delivered to suitable recipient cells in order to bring about an immune response (e. g., T-cell activation) in the treated subject.

### Particulate Compositions

Alternatively, the polynucleotide, adjuvant or compound can be formulated as a particulate composition. This can be carried out using standard pharmaceutical formulation chemistries and methodologies all of which are readily available to the reasonably skilled artisan.

The particulate composition will comprise an acceptable excipient or vehicle. Auxiliary substances, such as wetting or emulsifying agents, pH buffering substances, and the like, may be present in the excipient or vehicle. These excipients, vehicles and auxiliary substances are generally pharmaceutical agents that do not themselves induce an immune response in the individual receiving the composition, and which may be administered without undue toxicity. Pharmaceutically acceptable excipients include, but are not limited to, liquids such as water, saline, polyethyleneglycol, hyaluronic acid, glycerol and ethanol. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like.

Examples of suitable carriers that also act as stabilizers for peptides include, without limitation, pharmaceutical grades of dextrose, sucrose, lactose, trehalose, mannitol, sorbitol, inositol, dextran, and the like. Other suitable carriers include, again without limitation, starch, cellulose, sodium or calcium phosphates, citric acid, tartaric acid, glycine, high molecular weight polyethylene glycols (PEGs), and combination thereof. A thorough discussion of pharmaceutically acceptable excipients, carriers, stabilizers and other auxiliary substances is available in REMINGTONS PHARMACEUTICAL SCIENCES (Mack Pub. Co., N. J. 1991).

The formulated compositions will include an amount of the polynucleotide or compound which is sufficient to mount an immunological response, as defined above. An appropriate effective amount can be readily determined by one of skill in the art. Such an amount will fall in a relatively broad range, generally within the range of about 0.1 ug to 25 mg or more, and specific suitable amounts can be determined through routine trials. If an adjuvant is included in the composition, or the methods are used to provide a particulate adjuvant composition, the adjuvant will be present in a suitable amount as described above. The compositions are then prepared as particles using standard techniques, such as by simple evaporation (air drying), vacuum drying, spray drying, freeze drying (lyophilization), spray-freeze drying, spray coating, precipitation, supercritical fluid particle formation, and the like. If desired, the resultant particles can be densified using the techniques described in commonly owned International Publication No. WO 97/48485.

These methods can be used to obtain polynucleotide particles having a size ranging from about 0.1 to about 250 um, preferably about 10 to about 150 um, and most preferably about 20 to about 60 um; and a particle density ranging from about 0.1 to about 25 g/cm3, and a bulk density of about 0.5 to about 3.0 g/cm3, or greater.

Similarly, particles of adjuvants or the compound having a size ranging from about 0.1 to about 250 um, preferably about 0.1 to about 150 um, and most preferably about 20 to about 60 um ; a particle density ranging from about 0.1 to about 25 g/cm3, and a bulk density of preferably about 0.5 to about 3.0 g/cm3, and most preferably about 0.8 to about 1.5 g/cm3 can be obtained.

### Administration of Particulate Compositions

Following formation, the particulate composition (e. g., powder) can be delivered transdermally to vertebrate tissue using a suitable transdermal particle delivery technique. Various particle delivery devices suitable for administering the substance of interest are known in the art, and will find use in the practice of the invention. A particularly preferred transdermal particle delivery system employs a needleless syringe to fire solid particles in controlled doses into and through intact skin and tissue. See, e. g., U. S. Patent No. 5,630,796 to Bellhouse et al. which describes a needleless syringe (also known as "the PowderJect particle delivery device"). Other needleless syringe configurations are known in the art and are described herein.

The particulate compositions can then be administered using a transdermal delivery technique. Preferably, the particulate compositions will be delivered via a powder injection method, e. g., delivered from a needleless syringe such as those described in commonly owned International Publication Nos. WO 94/24263, WO 96/04947, WO 96/12513, and WO 96/20022. Delivery of particles from such particle delivery devices is practiced with particles having an approximate size generally ranging from 0.1 to 250 um, preferably ranging from about 10-70 um. Particles larger than about 250 um can also be delivered from the devices, with the upper limitation being the point at which the size of the particles would cause untoward damage to the skin cells. The actual distance which the delivered particles will penetrate a target surface depends upon particle size (e. g., the nominal particle diameter assuming a roughly spherical particle geometry), particle density, the initial velocity at which the particle impacts the surface, and the density and kinematic viscosity of the targeted skin tissue. In this regard, optimal particle densities for use in needleless injection generally range between about 0.1 and 25 g/cm3, preferably between about 0.9 and 1.5 g/cm3, and injection velocities generally range between about 100 and 3,000 m/sec, or greater. With appropriate gas pressure, particles having an average diameter of 10-70 um can be accelerated through the nozzle at velocities approaching the supersonic speeds of a driving gas flow.

If desired, these particle delivery devices (e. g., a needleless syringe) can be provided in a preloaded condition containing a suitable dosage of the particles comprising the antigen of interest and/or the selected adjuvant. The loaded syringe can be packaged in a hermetically sealed container, which may further be labeled as described above.

Compositions containing a therapeutically effective amount of the powdered molecules described herein can be delivered to any suitable target tissue via the above-described particle delivery devices. For example, the compositions can be delivered to muscle, skin, brain, lung, liver, spleen, bone marrow, thymus, heart, lymph, blood, bone cartilage, pancreas, kidney, gall bladder, stomach, intestine, testis, ovary, uterus, rectum, nervous system, eye, gland and connective tissues. For nucleic acid molecules, delivery is preferably to, and the molecules expressed in, terminally differentiated cells; however, the molecules can also be delivered to non-differentiated, or partially differentiated cells such as stem cells of blood and skin fibroblasts.

The powdered compositions are administered to the subject to be treated in a manner compatible with the dosage formulation, and in an amount that will be prophylactically and/or therapeutically effective. The amount of the composition to be delivered, generally in the range of from 0.5 ug/kg to 100 ug/kg of nucleic acid molecule per dose, depends on the subject to be treated.

Doses may be as low as 0.5 ug for 50 kg subject, or approximately 0.01 ug/kg. Doses for other pharmaceuticals, such as physiological active peptides and proteins, generally range from about 0.1 ug to about 20 mg, preferably 10 ug to about 3 mg. The exact amount necessary will vary depending on the age and general condition of the individual to be treated, the severity of the condition being treated, the particular preparation delivered, the site of administration, as well as other factors. An appropriate effective amount can be readily determined by one of skill in the art.

Thus, a"therapeutically effective amount"of the present particulate compositions will be sufficient to bring about treatment or prevention of disease or condition symptoms, and will fall in a relatively broad range that can be determined through routine trials.

### Preferred formulations for delivery of the compound

The compound is administered in the form of a pharmaceutical formulation which is suitable for the topical or transdermal delivery of drugs. The formulation will comprise a therapeutically effective amount of the compound and generally also a pharmaceutically acceptable vehicle. Typically, in the formulation, the compound is present in an amount of about 0.05 to 20 per cent, preferably 0.5 percent to about 10 percent, by weight based on the total weight of said formulation. In a preferred embodiment the formulation contains about 2 to about 7 per by weight of the compound, for example about 5 per cent.

Typically the formulation is in the form of a cream, ointment or adhesive coating (such as a pressure sensitive adhesive coating or adhesive-coated sheet material). The formulation may additionally comprise substances that enhance skin penetration of drugs.

The formulation is preferably substantially non-irritating. Such a formulation will not cause unacceptable skin irritation in conventional repeat skin irritation tests in albino rabbits such as that described in Draize et al., "Appraisal of the Safety of Chemicals in Food, Drugs and Cosmetics", prepared by the Division of Pharmacology of the Food and Drug Administration, published originally in 1959 by the Association of Food and Drug Officials of the United States, Topeka, Kans. (2nd printing 1965).

A fatty acid such as isostearic acid, oleic acid or a mixture thereof is incorporated into a formulation of the invention. The total amount of fatty acid present in a formulation is preferably about 3 percent to about 45 percent, preferably about 5 per cent to about 25 per cent by weight based on the total weight of formulation.

The formulation is preferably a cream according containing the compound of the invention and optionally also fatty acid. The cream generally comprises an oil phase and a water phase in admixture.

Optionally, the formulation (in particular when it is a cream or ointment) can contain one or more emollients, emulsifiers, thickeners and/or preservatives.

The emollients are typically long chain alcohols, such as cetyl alcohol, stearyl alcohol and cetearyl alcohol; hydrocarbons such as petrolatum and light mineral oil; or acetylated lanolin. The total amount of emollient in the formulation is preferably about 5 percent to about 30 percent, and more preferably about 5 percent to about 10 percent by weight based on the total weight of the formulation. The emulsifier is typically a nonionic surface active agent, e.g., polysorbate 60 (available from ICI Americas), sorbitan monostearate, polyglyceryl-4 oleate, and polyoxyethylene(4)lauryl ether or trivalent cationic. Generally the total amount of emulsifier is preferably about 2 percent to about 14 percent, and more preferably about 2 percent to about 6 percent by weight based on the total weight of the formulation.

Pharmaceutically acceptable thickeners, such as Veegum.TM.K (available from R. T. Vanderbilt Company, Inc.), and long chain alcohols (i.e. cetyl alcohol, stearyl alcohol or cetearyl alcohol) can be used.. The total amount of thickener present is preferably about 3 percent to about 12 percent by weight based on the total weight of the formulation.

Preservatives such as methylparaben, propylparaben and benzyl alcohol can be present in the formulation. The appropriate amount of such preservative(s) is known to those skilled in the art.

Optionally, an additional solubilizing agent such as benzyl alcohol, lactic acid, acetic acid, stearic acid or hydrochloric acid can be included in the formulation. If an additional solubilizing agent is used, the amount present is preferably about 1 percent to about 12 percent by weight based on the total weight of the cream.

Optionally, the formulation can contain a humectant such as glycerin and skin penetration enhancers such as butyl stearate.

It is known to those skilled in the art that a single ingredient can perform more than one function in a cream, i.e., cetyl alcohol can serve both as an emollient and as a thickener.

Generally, a cream consists of an oil phase and a water phase mixed together to form an emulsion. Preferably, the amount of water present in a cream of the invention is about 45 percent to about 85 percent by weight based on the total weight of the cream.

Where the formulation is an ointment a pharmaceutically acceptable ointment base such as petrolatum or polyethylene glycol 400 (available from Union Carbide) in combination with polyethylene glycol 3350 (available from Union Carbide) can be used. The amount of ointment base present in an ointment of the invention is preferably about 60 percent to about 95 percent by weight based on the total weight of ointment.

In a preferred embodiment the formulation is a cream which comprises an oil-in-water cream base comprising isotearic acid, cetyl alcohol, stearyl alcohol, white petrolatum, polysorbate 60, sorbiton monostearate, glycerin, xanthum gum, purified water, benzyl alcohol, methylparaban and propyl-paraban. Such a cream may be in the form of Aldara imiquimod cream which contains 5% imiquimod.

In another preferred embodiment the formulation comprises about 1 percent compound of the invention, about 10 percent of said isostearic acid, about 2 percent benzyl alcohol, about 2.2 percent cetyl alcohol, about 3.1 percent stearyl alcohol, about 2.55 percent polysorbate 60, about 0.45 percent sorbitan monostearate, about 2 percent glycerin, about 0.2 percent methylparaben, about 0.02 percent propylparaben and about 76.48 percent purified water, all percentages being based on the total weight of said formulation.

In another embodiment the formulation comprises about 1 percent compound of the invention, about 10 percent of said isostearic acid, about 6 percent cetearyl alcohol, about 2.55 percent polysorbate 60, about 0.45 percent sorbitan monostearate, about 2 percent glycerin, about 0.2 percent methylparaben, about 0.02 percent propylparaben and about 77.78 percent purified water, all percentages being based on the total weight of said formulation.

A further embodiment comprises about 1 percent compound, about 10 percent of said isostearic acid about 2 percent benzyl alcohol, about 1.7 percent cetyl alcohol, about 2.3 percent stearyl alcohol, about 2.55 percent polysorbate 60, about 0.45 percent sorbitan monostearate, about 2 percent glycerin, about 0.2 percent methylparaben, about 0.02 percent propylparaben and about 77.78 percent purified water, all percentages being based on the total weight of said formulation.

The formulation may comprise about 5 percent compound, about 25 percent of said isostearic acid, about 2 percent benzyl alcohol, about 2.2 percent cetyl alcohol, about 3.1 percent stearyl alcohol, about 3 percent petrolatum, about 3.4 percent polysorbate 60, about 0.6 percent sorbitan monostearate, about 2 percent glycerin, about 0.2 percent methylparaben, about 0.02 percent propylparaben and about 53.48 percent purified water, all percentages being based on the total weight of said formulation.

Alternatively the formulation may comprise about 1 percent compound, about 5 percent of said isostearic acid, about 15 percent petrolatum, about 12.8 percent light mineral oil, about 8 percent aluminum stearate, about 4 percent cetyl alcohol, about 3 percent polyglyceryl-4 oleate, about 1 percent acetylated lanolin, about 0.063 percent propylparaben, about 1 percent Veegum K, about 0.12 percent methylparaben and about 49.02 percent purified water, all percentages being based on the total weight of said formulation.

A pressure-sensitive adhesive composition generally comprises the compound, fatty acid, and a pressure sensitive adhesive polymer. The amount of the compound present in a pressure sensitive adhesive composition is preferably about 0.5 percent to about 9 percent by weight, and more preferably about 3 percent to about 7 percent by weight based on the total weight of the adhesive composition. The amount of fatty acid present is preferably about 10 percent to about 40 percent by weight, more preferably about 15 percent to about 30 percent by weight, and most preferably about 20 percent to about 30 percent by weight, based on the total weight of the adhesive composition.

Preferably, the adhesive polymer utilized in a pressure sensitive adhesive composition is substantially chemically inert to the compound. The adhesive polymer is preferably present in an amount of about 55 percent to about 85 percent by weight based on the total weight of the composition. Suitable adhesive polymers include acrylic adhesives that contain, as a major constituent (i.e., at least about 80 percent by weight of all monomers in the polymer), a hydrophobic monomeric acrylic or methacrylic acid ester of an alkyl alcohol, the alkyl alcohol containing 4 to 10 carbon atoms. Examples of suitable monomers are those discussed below in connection with the "A Monomer". These adhesive polymers can further contain minor amounts of other monomers such as the "B Monomers" listed below.

Preferred adhesives include acrylic pressure-sensitive adhesive copolymers containing A and B Monomers as follows: Monomer A is a hydrophobic monomeric acrylic or methacrylic acid ester of an alkyl alcohol, the alkyl alcohol containing 4 to 10 carbon atoms, preferably 6 to 10 carbon atoms, more preferably 6 to 8 carbon atoms, and most preferably 8 carbon atoms. Examples of suitable A Monomers are n-butyl, n-pentyl, n-hexyl, isoheptyl, n-nonyl, n-decyl, isohexyl, 2-ethyloctyl, isooctyl and 2-ethylhexyl acrylates. The most preferred A Monomer is isooctyl acrylate.

Monomer B is a reinforcing monomer selected from the group consisting of acrylic acid; methacrylic acid; alkyl acrylates and methacrylates containing 1 to to 3 carbon atoms in the alkyl group; acrylamide; methacrylamide; lower alkyl-substituted acrylamides (i.e., the alkyl group containing 1 to 4 carbon atoms) such as tertiary-butyl acrylamide; diacetone acrylamide; n-vinyl-2-pyrrolidone; vinyl ethers such as vinyl tertiary-butyl ether; substituted ethylenes such as derivatives of maleic anhydride, dimethyl itaconate and monoethyl formate and vinyl perfluoro-n-butyrate. The preferred B Monomers are acrylic acid, methacrylic acid, the above-described alkyl acrylates and methacrylates, acrylamide, methacrylamide, and the above-described lower alkyl substituted acrylamides. The most preferred B Monomer is acrylamide.

In one embodiment of a pressure-sensitive adhesive composition, the pressure-sensitive adhesive copolymer containing A and B Monomers as set forth above preferably contains the A Monomer in an amount by weight of about 80 percent to about 98 percent of the total weight of all monomers in the copolymer. The A Monomer is more preferably present in an amount by weight of about 88 percent to about 98 percent, and is most preferably present in an amount by weight of about 91 percent to about 98 percent. The B Monomer in such a copolymer is preferably present in the pressure-sensitive adhesive copolymer in an amount by weight of about 2 percent to about 20 percent, more preferably about 2 percent to about 12 percent, and most preferably 2 to 9 percent of the total weight of the monomers in the copolymer.

In another embodiment of a pressure-sensitive adhesive composition, the adhesive copolymer comprises about 60 to about 80 percent by weight (and preferably about 70 to about 80 percent by weight) of the above-mentioned hydrophobic monomeric acrylic or methacrylic acid ester of an alkyl alcohol (i.e., Monomer A described above) based on the total weight of all monomers in the copolymer; about 4 to about 9 percent by weight based on the total weight of all monomers in the copolymer of a reinforcing monomer selected from the group consisting of acrylic acid, methacrylic acid, an alkyl acrylate or methacrylate containing 1 to 3 carbon atoms in the alkyl group, acrylamide, methacrylamide, a lower alkyl-substituted acrylamide, diacetone acrylamide and N-vinyl-2-pyrrolidone; and about 15 to about 35 percent by weight (and preferably about 15 to about 25 percent by weight) of vinyl acetate based on the total weight of all monomers in the copolymer. In this embodiment the preferred acrylic or methacrylic acid ester is isooctyl acrylate and the preferred reinforcing monomer is acrylamide.

The above described adhesive copolymers are known, and methods of preparation therefor are well known to those skilled in the art, having been described for example, in U.S. Pat. No. 24,906 (Ulrich). The polymerization reaction can be carried out using a free radical initiator such as an organic peroxide (e.g., benzoylperoxide) or an organic azo compound (e.g., 2,2'-azobis(2,4-dimethylpentanenitrile), available under the trade designation "Vazo 52" from DuPont).

Since pressure-sensitive adhesives such as those described above are inherently rubbery and tacky and are suitably heat and light stable, there is no need to add tackifiers or stabilizers. However, such can be added if desired.

Optionally, a pressure-sensitive adhesive composition (or indeed any other composition described herein) can also contain one or more skin penetration enhancers such as glyceryl monolaurate, ethyl oleate, isopropyl myristate, diisopropyl adipate and N,N-dimethyldodecylamine-N-oxide, either as a single ingredient or as a combination of two or more ingredients. The skin penetration enhancer(s) preferably form a substantially homogeneous mixture with the pressure sensitive adhesive polymer or copolymer. The total amount of skin penetration enhancer(s) present in a pressure sensitive adhesive composition of the invention is preferably about 3 percent to about 25 percent by weight, more preferably about 3 percent to about 10 percent by weight based on the total weight of the adhesive composition.

When the skin penetration enhancer is a single ingredient, it is preferably a skin penetration enhancer such as isopropyl myristate, diisopropyl adipate, ethyl oleate, or glyceryl monolaurate. When a combination skin penetration enhancer is used, it is preferably a combination such as: ethyl oleate with glyceryl monolaurate; ethyl oleate with N,N-dimethyldodecylamine-N-oxide; glyceryl monolaurate with N,N-dimethyldodecylamine-N-oxide; and ethyl oleate with both glyceryl monolaurate and N,N-dimethyldodecylamine-N-oxide.

The pressure-sensitive adhesive compositions described above are preferably coated onto one surface of a suitable backing of sheet material, such as a film, to form a pressure-sensitive adhesive coated sheet material. A pressure-sensitive adhesive coated sheet material can be prepared by knife coating a suitable release liner to a predetermined uniform thickness with a wet adhesive formulation. This adhesive coated release liner is then dried and laminated onto a backing using conventional methods. Suitable release liners include conventional release liners comprising a known sheet material, such as a polyester web, a polyethylene web, or a polystyrene web, or polyethylene-coated paper, coated with a suitable silicone-type coating such as that available under the trade designation Daubert 164Z, from Daubert Co. The backing can be occlusive, non-occlusive or a breathable film as desired. The backing can be any of the conventional materials for pressure-sensitive adhesive tapes, such as polyethylene, particularly low density polyethylene, linear low density polyethylene, high density polyethylene, randomly-oriented nylon fibers, polypropylene, ethylenevinylacetate copolymer, polyurethane, rayon and the like. Backings that are layered, such as polyethylene-aluminum-polyethylene composites are also suitable. The backing should be substantially non-reactive with the ingredients of the adhesive coating. The presently preferred backing is low density polyethylene.

The pressure-sensitive adhesive coated sheet material can be made in the form of an article such as a tape, a patch, a sheet, a dressing or any other form known to those skilled in the art.

Preferably, an article in the form of a patch is made from an adhesive coated sheet material of the invention and applied to the skin of a mammal. The patch is replaced as necessary with a fresh patch to maintain the particular desired therapeutic effect of the compound of the invention.

Formulations which are suitable for use in the invention are described in the prior art, for example in US-A-5,238,944 (which discloses preferred formulations for use in the method of the invention), US-A-4,689,338, U.S. Pat. No. 4,751,087 (discloses the use of a combination of ethyl oleate and glyceryl monolaurate as a skin penetration enhancer for nitroglycerine, with all three components being contained in the adhesive layer of a transdermal patch), U.S. Pat. No. 4,411,893 (discloses the use of N,N-dimethyldodecylamine-N-oxide as a skin penetration enhancer in aqueous systems), U.S. Pat. No. 4,722,941 (discloses readily absorbable pharmaceutical compositions that comprise a pharmacologically active agent distributed in a vehicle comprising an absorption-enhancing amount of at least one fatty acid containing 6 to 12 carbon atoms and optionally a fatty acid monoglyceride), U.S. Pat. No. 4,746,515 (discloses a method of using glyceryl monolaurate to enhance the transdermal flux of a transdermally deliverable drug through intact skin).

Below are examples of specific embodiments for carrying out the present invention. The examples are offered for illustrative purposes only, and are not intended to limit the scope of the present invention in any way.

### Examples

### Materials and Method

### Plasmids

For immunization with the Hepatitis B surface antigen (HBsAg) a plasmid containing the HCMV promoter/enhancer was used to drive expression of the HBsAg. For one experiment we also employed a plasmid in which the keratin 14 promoter was used instead of the HCMV promoter. This promoter is expected to have a more restricted pattern of expression and be expressed only in the skin unlike the HCMV promoter that is expressed in many cell types.

For immunization with HSV-2 antigens in some cases single gene plasmids expressing either the gD or gB proteins using the HCMV promoter were used. The single gene plasmids were created by amplification of genomic sequences by PCR and insertion of the PCR fragment into the pTARGET expression vector. In some instances genomic fragments of HSV-2 were used for immunization which we call subgenomic vaccines (SV). These do not use the HCMV promoter but rather use the native promoters from the genes present in the fragment. The genomic fragments are cloned in the SuperCos backbone from Stratagene. The subgenomic vaccine has a genomic segment of approximately 36,000 bases containing nucleotides 110,931-147,530 of the genome.

In some experiments plasmids were used that contained the Cholera toxin (CT) genes A and B subunits. Another plasmid used expressed the HSP70 gene. This gene was cloned from a RT-PCR reaction of RNA obtained from mouse splenocytes, and was cloned into the pTARGET vector.

All DNA constructs were purified from bacterial extracts using purification kits (Qiagen) and purity was assessed by agarose gel electrophoresis of whole or digested plasmids and by determining the A260/A280 ratios.

### Topical Adjuvants.

Imiquimod was obtained in the form of Aldara cream from a prescription. To apply, the cream was rubbed onto the abdomens of mice that had been clipped using a cotton swab. The cream is a 5% solution. About 20 ul was used, and so approximately 1 mg was given to each mouse.

### Preparation of DNA vaccines.

Precipitation of DNA onto gold particles was achieved using standard procedures for the calcium/spermidine formulation of DNA vaccines. DNA was mixed with 2 micron gold particles in a small centrifuge tube containing 300 ml of 50 mM spermidine. The amount of DNA added is 2 ug per mg gold particles and typically batches of 26 mg gold (52 ug of DNA) were made. The DNA was precipitated onto gold by the addition of a 1/10 volume of 10% CaCl₂ during continuous agitation of the tube on a rotary mixer. DNA-gold complexes were washed three times with absolute ethanol then loaded into Tefzel tubing, dried and cut into 0.5 inch segments for use in the XR-1 device.

Instances where more than one DNA was used was done by first mixing the DNA constructs then precipitation onto gold. This puts the DNAs onto the same particle.

For immunization, DNA vaccines were delivered by the XR-1 device into the abdomen of Balb/C mice. A single shot was given for the immunization. Some experiments employed a prime only, and others had a prime and a boost at 4 weeks. Samples were collected from animals two weeks after the final immunization.

### Antibody ELISA

Serum samples were assayed for antibodies using an ELISA assay. Falcon Pro Bind microtiter plates were coated overnight at 4 °C with antigen in PBS (phosphate buffered saline, BioWhittaker). For HBsAg ELISA the antigen was purified HBsAg (BioDesign) at 0.1 ug per well, and for HSV ELISAs the antigen was 5 ug per well of an infected cell extract (Advanced Biotechnolgies Incorporated). The plates were blocked for 1 hour at RT with 5% dry milk/PBS then washed 3X with wash buffer (10 mM Tris Buffered saline, 0.1% Brij-35) and serum samples diluted in dilution buffer (2% dry milk/PBS/0.05 % Tween 20) were added to the plate and then incubated for 2 hours at RT.

Plates were washed 3X and a biotinylated goat anti-mouse antibody (Southern Biotechnology) diluted 1:8000 in dilution buffer was added to the plate and incubated for 1 hr at RT. Following the incubation, plates were washed 3X, then a Streptavidin-Horseradish peroxidase conjugate (Southern Biotechnology) diluted 1:8000 in PBS was added and the plate incubated a further 1 hr at RT. Plates were washed 3X, then substrate solution added (BioRad) and the reaction was stopped with 1N H₂SO₄. Optical density was read at 450 nm. Endpoint titers for HBsAg were calculated by comparison of the samples with a standard of known titer.

### Cell Culture

Single cell suspensions were obtained from mouse spleens. Spleens were squeezed through a mesh to produce a single cell suspension and cells were then sedimented, and treated with ACK buffer (Bio Whittaker, Walkersville MD) to lyse red blood cells. The cells were then washed twice in RPMI 1640 media supplemented with HEPES, 1 % glutamine (Bio Whittaker), and 5% heat inactivated fetal calf serum (FCS, Harlan, Indianapolis IN). Cells were counted, and resuspended to an appropriate concentration in "Total" media consisting of RPMI 1640 with HEPES and 1% glutamine, supplemented with 5% heat inactivated FCS, 50 mM mercaptoethanol (Gibco-BRL, Long Island NY), gentamycin (Gibco-BRL), 1 mM MEM sodium pyruvate (Gibco-BRL) and MEM non-essential amino acids (Sigma, St, Louis MO). Cell suspensions were then utilized in various immunoassays. For CD8 specific assays cells were cultured *in vitro* in the presence of a peptide corresponding to a known CD8 epitopes. For HBsAg in BALB/C mice the sequence of the peptide was IPQSLDSWWTSL (QCB Inc). For HSV CD8 responses in Balb/C mice the HGPSLYRTF peptide found in ICP27 was used. Peptides were made up in DMSO (10 mg/ml) and diluted to 10 ug/ml in culture medium.

### ELISPOTs

For IFN-g ELISPOTs assays Millipore Multiscreen membrane filtration plates were coated with 50 ul of 15 ug/ml anti-IFN-g antiserum (Pharmingen) in sterile 0.1M carbonate buffer pH 9.6, overnight at 4 °C. Plates were washed 6X with sterile PBS and then blocked with tissue culture medium containing 10% fetal bovine serum (FBS) for 1-2 hr at RT. The medium was removed and spleen cells dispensed into the wells with a total of 1X10⁶ cells per well. For wells in which less than 1X10⁶ cells from immunized animals was added, cells from naïve animals were used to bring the total to 1X10⁶. Cells were incubated overnight in a tissue culture incubator in the presence of the peptide as described above. Plates were washed 2X with PBS and 1X with distilled water. This was followed with 3 washes with PBS. Biotinylated anti IFN-g monoclonal antibody (Pharmingen) was added to the plate (50 ul of a 1 ug/ml solution in PBS) and incubated for 2 hr at RT. Plates were washed 6X with PBS then 50 ul of a Streptavidin Alkaline phosphatase conjugate (1:1000 in PBS, Pharmingen) was added and incubated for 2 hr at RT. Plates were washed 6X with PBS and the color substrate (BioRad) was added and the reaction was allowed to proceed until dark spots appeared. The reaction was stopped by washing with water 3X. Plates were air dried and spots counted under a microscope.

### IFN-g ELISA

For the CD8 IFN-g ELISA cells were cultured overnight in round bottom 96 well tissue culture plates in the presence of the peptide. Samples of the supernatant were taken and used for the determination of IFN-g levels. High binding plates (Costar) were coated with 100 ul of 0.5 ug/ml of anti-mouse IFN-g antibody (Pharmingen)in bicarbonate buffer pH 9.6. Plates were blocked for 1 hr at RT with tissue culture medium containing 10% FBS then washed 3X with the TBS wash buffer. Supernatant samples obtained from cultured cells were diluted in tissue culture medium and loaded onto the plate and incubated for 2 hr at RT. Plates were washed 3X with wash buffer and a secondary antibody (0.5 ug/ml of biotinylated rat anti-mouse INF-g in PBS, Pharmingen) was added to the plates and incubated for 1 hr at RT. Plates were washed 3X, and a Streptavidin-horseradish peroxidase conjugate (1:2000 in PBS, Southern Biotechnology) was added for 1 hr at RT. Plates were washed 3X, then substrate solution added (BioRad) and the reaction was stopped with 1N H₂SO₄. Optical density was read at 450 nm.

When IFN-g ELISAs were carried out on cells stimulated with the UV-inactivated HSV-2, the supernatants from the proliferation plates (described below) were used as a source of the experimental IFN-g. Otherwise the procedure was the same as described above.

### Proliferation assays.

For proliferation cells were plated at a concentration of 3 X 10⁵ cells/well in Costar 96 well plates (Coming Incorporated, Corning NY). UV-inactivated virus (original moi 2), or infected cell protein extracts (2 and 0.5 ug per well) was added to triplicate wells and the cells incubated at 37°C in a CO₂ atmosphere for 3 days. Then methyl-³H thymidine (NEN, Boston MD) was added (0.5 mC well) and cells were cultured for a further 18 hr before plates were processed. Stimulation indices were calculated by taking the average cpm of triplicate wells for cells stimulated by antigen, divided by the average of triplicate wells of cells cultured with medium only.

### Initial experiment to investigate potential adjuvant activity of imiquimod

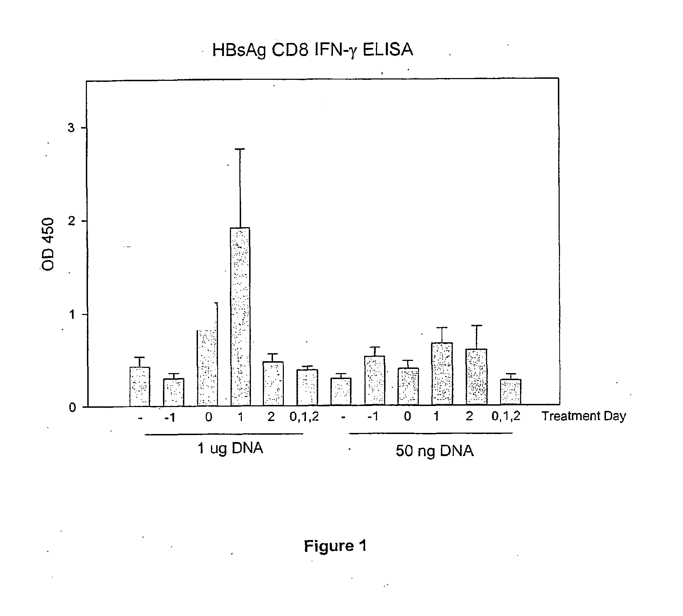

Mice were immunized with a DNA vaccine containing a HBsAg expressing plasmid using either a high dose (1ug) or low dose (50 ng) of vaccine. Treatment with Aldara 5% imiquimod cream was either 1 day before immunization (day -1), on the day of immunization (day 0), 1 day following or 2 days following immunization. One group was also treated for three days (day 0, 1 and 2). Mice were given a single dose of vaccine and were sacrificed 2 weeks later to measure immune responses. Results shown in Figure 1 are from the CD8 IFN-g ELISA. From this data there was an indication that the Aldara could boost cellular immune responses. Some effect was found at day 0 but day 1 appeared to be better although this was largely due to two strong responders. The values are the means from 4 individual mice.

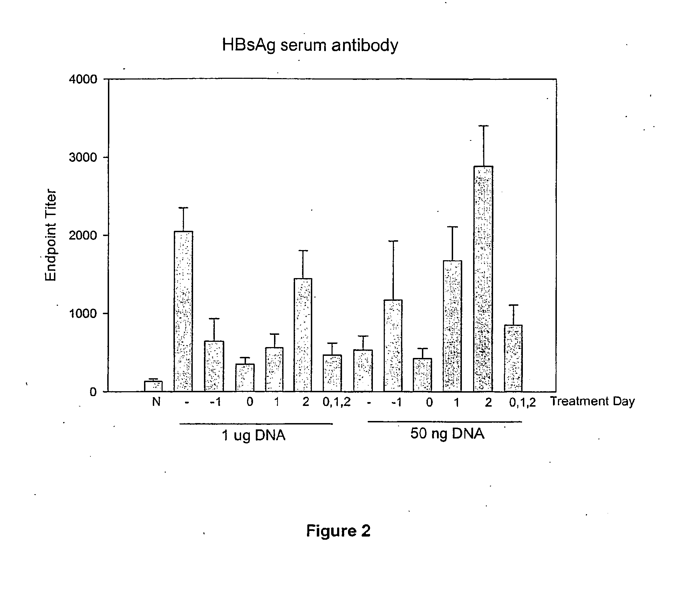

The serum antibody was also tested (Figure 2), but because these are only 2 weeks after prime they tended to be variable.

### Example 2.

### Imiquimod enhances cellular responses when applied one day after immunisation

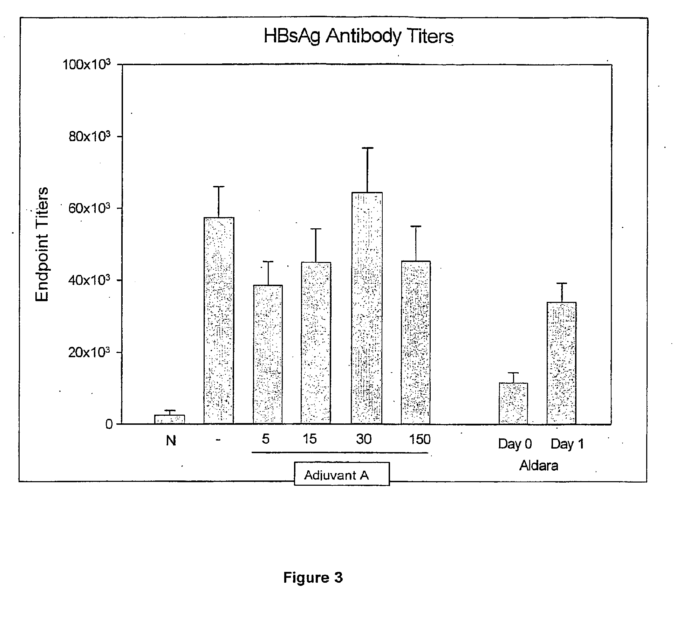

The experiment described in Example 1 was repeated. In addition various doses of another adjuvant (adjuvant A) were tested. The animals were given a prime and boost using a plasmid expressing the HBsAg gene from the HCMV promoter. Antibody levels were slightly affected by adjuvant A but in this instance the effect of imiquimod can be clearly seen and a strong reduction in antibody levels was found (Figure 3).

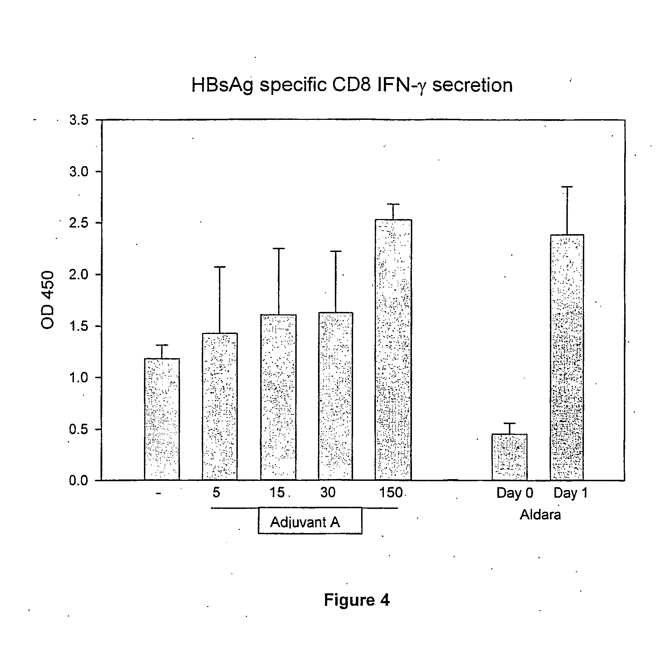

The cellular response shown by both the IFN-g ELISA and ELISPOTs indicated that the treatment with imiquimod enhanced the responses. The animals treated with Aldara one day after immunization showed again the strongest effect (Figure 4).

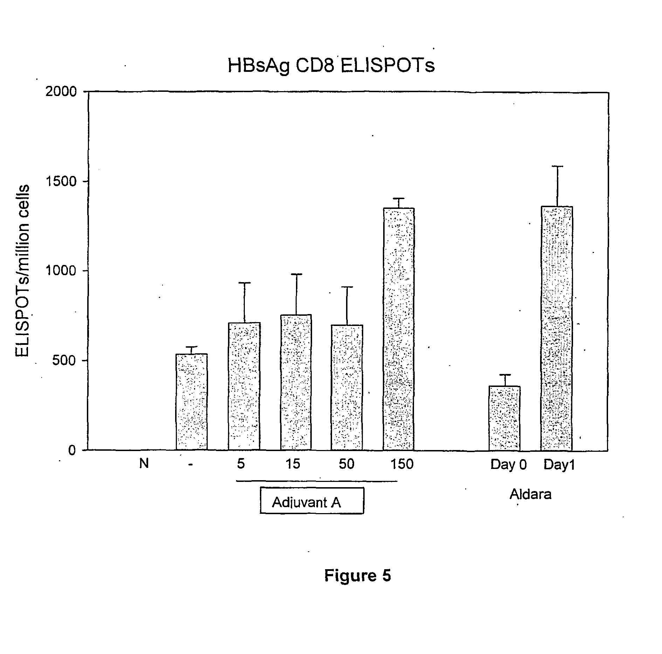

This is confirmed by the data shown in Figure 5. In all the following experiments the Aldara cream was given on Day 1 after immunization.

### Example 3.

### The effect of imiquimod on a variety of different boost and prime strategies.

This experiment includes a plasmid that expresses HBsAg from the keratin 14 promoter. This was used to try and alter the expression of the antigen in different cell types and see if this affects the immune response. Mice were immunized twice. Some were treated with Aldara cream at prime only, some were given Aldara only at boost and one group received Aldara at both prime and boost.

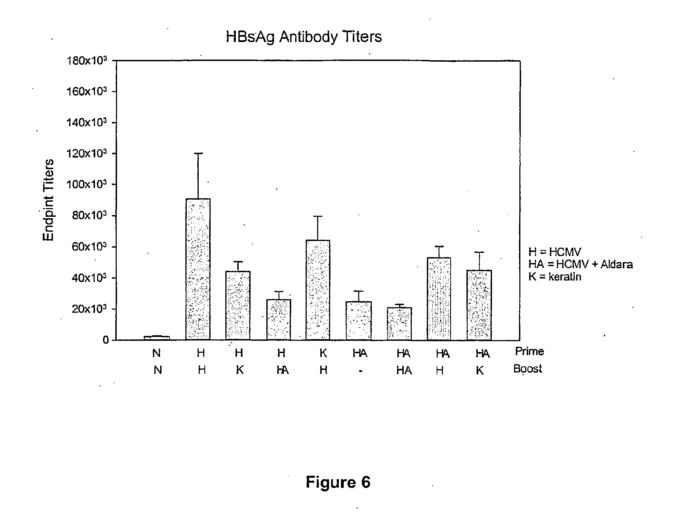

Results are shown in Figure 6. Antibody titers were not enhanced by the treatments and as often found they were in fact reduced. The most pronounced reduction for mice given two immunizations is the group given Aldara cream at prime and at boost. In the Figures the naïve animals are labeled as "N" and animals labeled as HA,- (sixty group) were given only a single immunization.

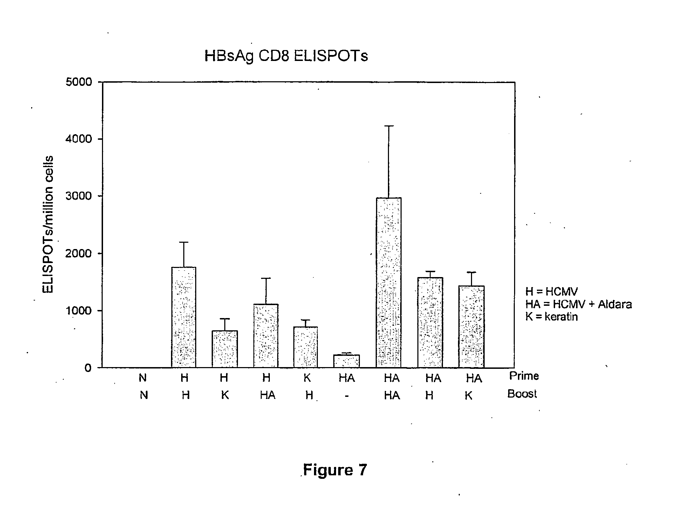

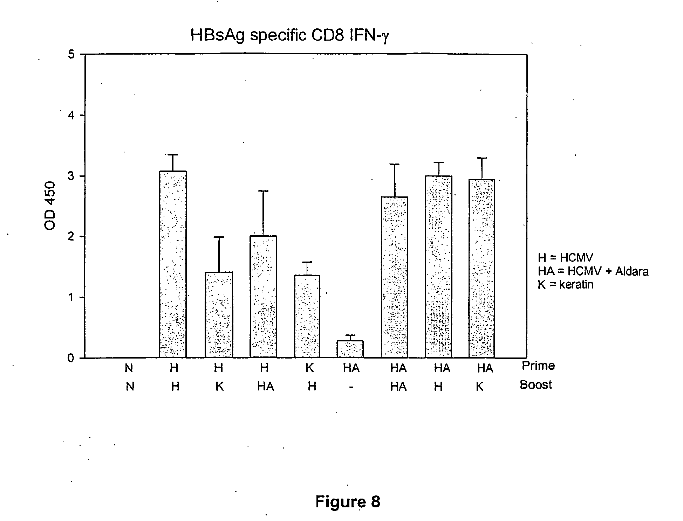

When cellular immune responses were measured (Figure 7 and 8) we did not get the expected pattern because the control group given two immunizations with HCMV plasmid (H,H) had responses considerably higher than found in the past (see previous experiment). The abnormally high controls made it impossible to interpret the Aldara effects although if one considers that the H,H control should be more like the H,K group beside it an effect of the Aldara can be extrapolated. Overall, it appears that the Aldara enhances cellular responses, and inhibits antibody responses.

### Example 4

### Using imiquimod to enhance responses to HSV-2 antigens

Three types of DNA vaccine used in this experiment. A single gene gD plasmid that expresses the full length glycoprotein which would be a membrane protein. A single gene gB plasmid that expresses a truncated form of the protein so that it is secreted from the cell. The third type of vaccine is a subgenomic vaccine that contains a genomic fragment of HSV-2. Within this fragment is the gene for gD, but not gB. Other genes of interest in this vaccine are the immediate early genes ICP0, 4 22 and 27. The adjuvants used were Aldara cream applied topically, the CT genes co-delivered on the same gold particles as the vaccine and another adjuvant (adjuvant B) given on day 0, 1 and 2.

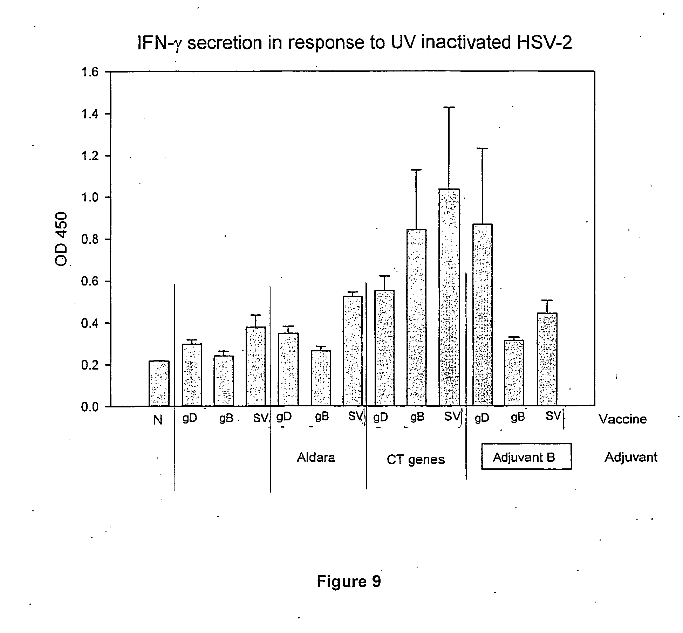

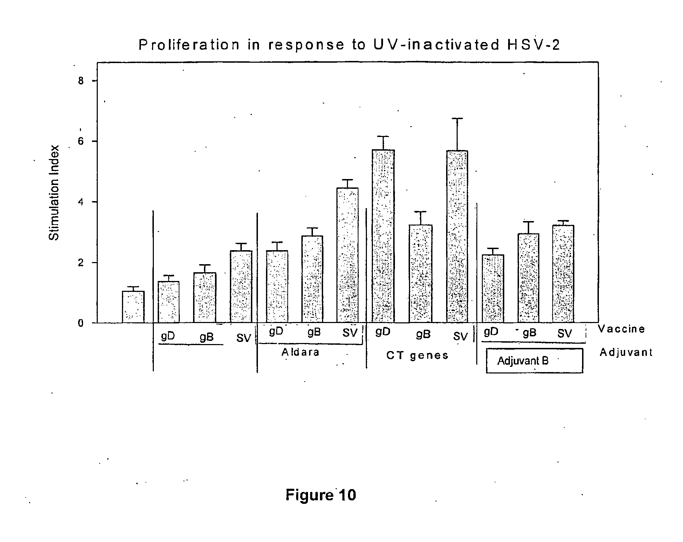

Cellular immune measures (Figures 9 and 10) show that the CT adjuvants are superior to the other adjuvants although some activity for the Aldara cream was apparent.

### Example 5

### Testing a panel of adjuvants

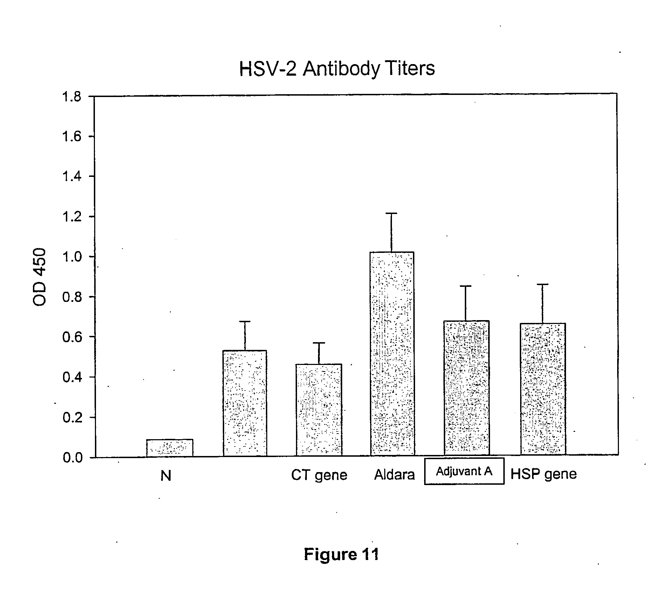

A panel of adjuvants were tested for activity with a HSV-2 vaccine which was the subgenomic vaccine. In this case adjuvant A and the HSP gene were used as well as Aldara cream and the CT genes. All adjuvants were given at a dose in which we had previously seen optimal effects in experiments with the HBsAg vaccines. The Aldara was applied one day after immunization, adjuvant A (150 ng) was given on the day of immunization, the CT and HSP genes were co-delivered with the subgenomic vaccine in 9:1 and 20:1 ratios of vaccine: adjuvant respctively. Animals were given a prime and boost. Antibody responses are typically low from the vaccine and none of the adjuvants showed a strong ability to boost these (Figure 11).

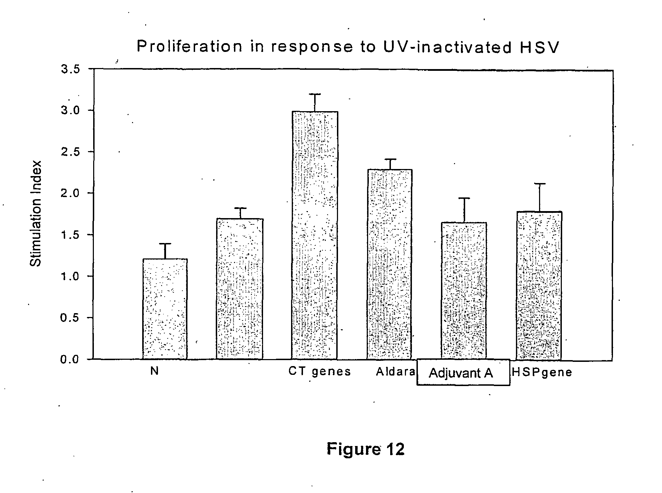

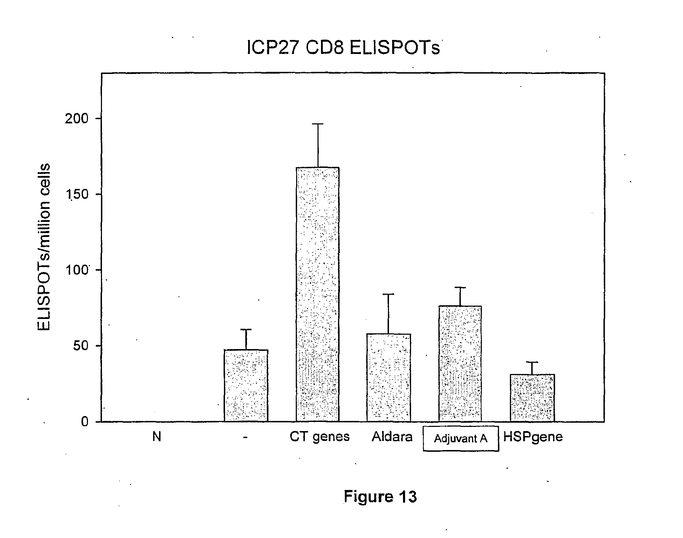

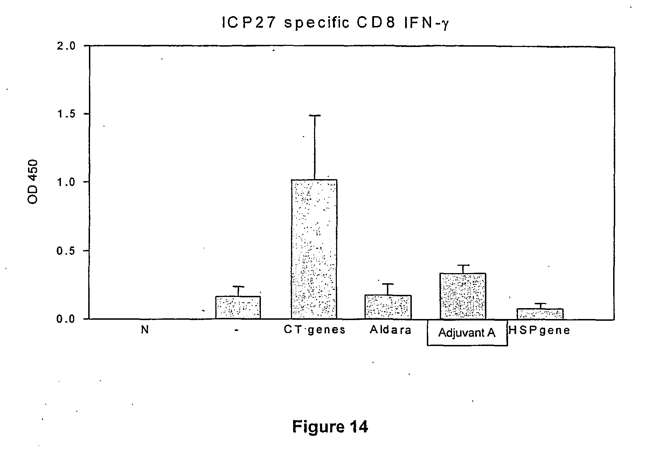

Cellular immune responses were measured by proliferations in response to UV-inactivated HSV-2 as well as specific CD8 responses to the ICP27 protein expressed from the vaccine (Figures 12 to 14).

### Example 6

### Testing antibody subclass

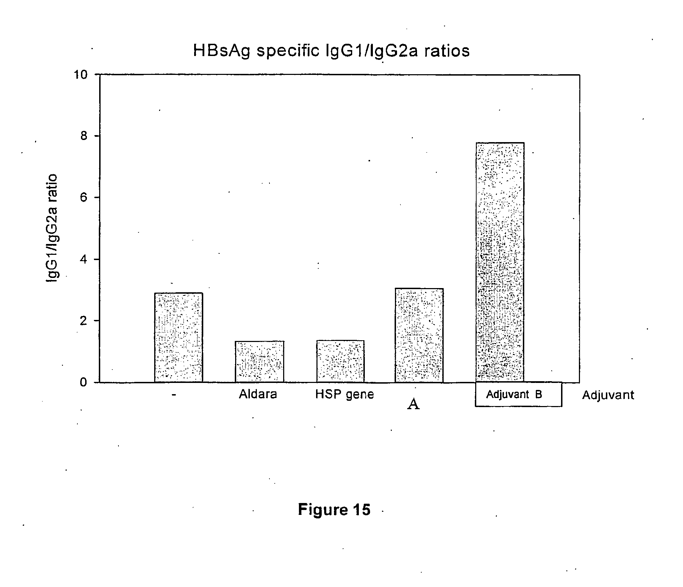

An ELISA was done on serum samples from different experiments to look at the subclass of the HBsAg specific antibody. Unadjuvanted responses from the DNA vaccine show a ratio of IgG1/IgG2a of about 3. A stronger cellular bias of an immune response is indicated by a stronger IgG2a response so the ratio would thus approach 1 or below. Of the adjuvants we have worked with the Aldara is one in which this ratio does drop indicating that it is able to bias the immune response towards a cellular response (Figure 15). PGE2 for example does not have the same effect overall even though it can enhance cellular responses as the Aldara does, but it does not seem to shift the immune response as much. This feature of imiquimod may be of importance when an antibody response is detrimental, or a greatly biased cellular response is needed.

### Example 7

### Further characterisation of the adjuvant activity of imiquimod

Two further experiments were performed to assess the efficacy of imiquimod (Aldara cream) as an adjuvant for particle-mediated immunization with DNA (PAID). Both experiments used a plasmid expressing HBV sAg and cAg as a test vaccine. The dosage of DNA, the formulation of Aldara (neat vs. diluted with control cream), and the time of Aldara administration were varied. Readouts included antibody titers and cytokine ELISPOTs to the two antigens. As expected from the results in previous Examples imiquimod primarily enhanced the IFN-g ELISPOT response with little effect on antibody titers and IL-4 ELISPOTs. The results also showed that delivering Aldara one or seven days after PMID was generally more effective than control cream or Aldara delivered at the time of PMID administration.

In order to test the efficacy of imiquimod as an adjuvant for PMID, HBV sAg-and cAg-encoding DNA was administered using an ND5.5 device and imiquimod was administered as Aldara cream. In the first experiment, different doses of DNA and times of administration of Aldara were tested (Table 1). In the second experiment, a single dose of HBV-encoding DNA (2 µg) was tested along with treatment with Aldara at 1 day post-PMID vaccination, diluted Aldara at the same time as PMID, and Aldara 1 week post-PMID (Table 2).

Groups of 5-10 Balb/c mice were vaccinated on the shaved abdomen with one shot each on day 0 (prime only) or days 0 and 28 (prime and boost). PMID was administered using an ND5.5 device with a payload of 1 mg Au and a 35 bar cylinder. Aldara was administered at the doses and times indicated in the tables above. Imiquimod was administered as Aldara cream (5% imiquimod) obtained from a pharmacist. In experiment one, a small, unmetered amount of Aldara was delivered at the indicated times using a cotton swab to rub the cream in. In experiment two, 20 ml of Aldara was measured and applied to the mice. The control cream was an over the counter hand cream which contained many of the same ingredients as the Aldara base cream. In the group where the Aldara was diluted, a 5:1 mix of control cream and Aldara was emulsified between two syringes using a three-way stopcock.

Mice were bled retro-orbitally at 4 weeks for prime / boost groups. Blood was collected by intracardiac puncture, and spleens were removed for cellular assays at sacrifice at either week 2 (prime only) or week 6 (boost + 2 weeks). Sera were analyzed for anti-sAg and anti-cAg antibodies using in-house ELISAs. Spleen cells from individual mice were stimulated with protein or peptide for g-IFN and IL-4 ELISPOT assays. Three sets of antigen were used: 1) intact cAg from Biodesign; 2) a library of overlapping sAg peptides (15 mers offset by 3 amino acids synthesized by Chiron); and 3) an immunodominant peptide from sAg (amino acids 28-39) known to bind the MHC class I molecule L^{d}. Specific spot-forming cells were calculated by subtracting the number of spots generated with cells cultured in medium alone.

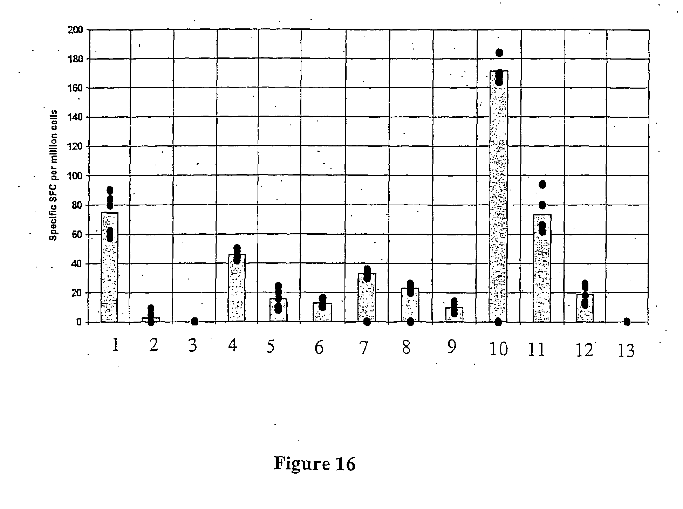

As observed previously, imiquimod did not enhance antibody responses to either HBV antigen (data not shown). In the first experiment, there was also no effect of imiquimod on IL-4 secretion, as measured by ELISPOT assay (also not shown). As shown in Figure 16, there was an enhancement of the frequency of cells which secreted g-IFN in response to antigen. The response to sAg peptide library (primarily CD4+ cells) is shown but comaparable responses were seen with a class 1-restricted peptide which stimulates CD8+ cells (not shown). At the low doses of HBV-encoding DNA, imiquimod enhances g-IFN secretion when administered at all three time points, although administration 24 hours post PMID is the most effective. At the highest dose of DNA, imiquimod administration is slightly inhibitory when delivered right before or right after but augments PMID when given 24 hours after.

In the second experiment, prime / boost regimens were attempted as well as lowering the dose of imiquimod by mixing Aldara with a control cream. It should be noted that as we had no way of measuring imiquimod, we are uncertain as to how effective the mixing was. In this study, the g-IFN ELISPOT responses of the mice given control cream was higher than normal after prime only. Therefore, there was no augmentation of the response by Aldara (data not shown). This is, unfortunately, not consistent with the first study. In the animals that were boosted, however, augmentation of the frequency of g-IFN secreting cells was seen when Aldara was administered at the following times: 24 hours after prime only; 24 hours after both the prime and boost; 24 hours after only the boost; and seven days after the boost. The largest effect occurred with the later doses of Aldara (1 and 7 days post-boost) (Figure 17).

As we have not tested ELISPOTs at longer time points after boost and Aldara treatment, we do not know if this effect is transient. As in experiment one, the CD8+ responses were similar to the CD4+ responses with the exception that Aldara delivered 24 hours after the boost only was not higher than when it was delivered after prime only: Unlike experiment one (prime only studies), augmentation of IL-4 ELISPOT responses was seen after boost, particularly with the group treated 7 days after the boost (data not shown).

These experiments show that imiquimod augments the frequency of g-IFN-secreting cells following PMID vaccination. The optimal time point for this effect seems to be about a day and upto 7 days after PMID.

**Table 1**

| Group | HBV sAg/cAg plasmid (µg) | Control Vector (µg) | Aldara (time relative to PMID) |
|---|---|---|---|
| 1 | 2 | 0 | 0 |
| 2 | 0.2 | 1.8 | 0 |
| 3 | 0.02 | 1.98 | 0 |
| 4 | 2 | 0 | Right before |
| 5 | 0.2 | 1.8 | Right before |
| 6 | 0.02 | 1.98 | Right before |
| 7 | 2 | 0 | Right after |
| 8 | 0.2 | 1.8 | Right after |
| 9 | 0.02 | 1.98 | Right after |
| 10 | 2 | 0 | One day after |
| 11 | 0.2 | 1.8 | One day after |
| 12 | 0.02 | 1.98 | One day after |
| 13 | 0 | 2 | 0 |

**Table 2**

| Group | Aldara Treatment (T=Time post vaccination) | Day of Sacrifice |
|---|---|---|
| 1 | Control cream T=24 hr | 14 post-prime |
| 2 | Aldara neat T=24 hr | 14 post-prime |
| 3 | Aldara diluted 1:5 T=0 | 14 post-prime |
| 4 | Aldara T=7 days | 14 post-prime |
| 5 | Naive | 14 post-prime |
| 6 | Control cream T=24 hr | 14 post-boost |
| 7 | Aldara neat T=24 hr at prime only | 14 post-boost |
| | | |
| 8 | Aldara neat T=24 hr at prime and boost | 14 post-boost |
| 9 | Aldara neat T=24 hr at boost only | 14 post-boost |
| 10 | Aldara T=7 days post-boost | 14 post-boost |
| 11 | Naive | 14 post-boost |

### Example 8

### Adjuvant activity of Resiquimod

Resiquimod is delivered topically to the abdominal skin of Balb/c mice on either day -2, -1, 0, 1, 2 or 3 relative to immunization on day 0. Immunization utilizes a DNA vaccine that expresses the Hepatitis B surface antigen so that cellular and antibody responses found after treatment with Resiquimod can be evaluated and compared to a wealth of historical data. The formulation consists of either a 0.05% cream, or a DMSO solution (approximately 5 mg/ml). Ideally both types of formula are tested. One half of the mice are sacrificed 7 days after immunization to measure cellular immune responses (CD8 ELISPOT). The remaining mice are bled 28 days after immunization, boosted with the same schedule as the priming immunization and then sacrificed 2 weeks later to test for antibody and cellular responses. Serum is used to test for antibody titer and to evaluation subclass distribution of IgG antibodies indicating the bias of the immune response.

At a time either before or after immunization the Resiquimod enhances immune responses to the DNA vaccines. The enhancement is likely occur when Resiquimod is delivered 1 day after immunization as found for Aldara, which is a similar compound. Most likely a boost in the cellular immune response is found. Resiquimod may have an ability to enhance antibody responses but this may not occur with the delivery site and schedule of administration that we employ. In theory, one time point (different than the enhancing point) may also find a suppressing effect of the Resiquimod, which may be exploited for other applications. The doses used appear to be in the range where activity of Resiquimod is found *in vivo,* and further refinement of the method will optimize dosing and formulation of the Resiquimod.

### Materials and Methods

### Plasmids

For immunization DNA vaccine plasmids with a well-known immune response are employed. Plasmid WRG7128 is a likely choice which expresses the Hepatitis B surface antigen (HBsAg) using the HCMV promoter/enhancer and generates a cellular and antibody response.

### Topical Adjuvants

Resiquimod is formulated as either a 0.05% cream, or a 5 mg/ml solution in DMSO.. To apply the cream, it is rubbed onto the abdomens of mice using a cotton swab. Approximately 20 ml of cream is given by this method. The DMSO solution is spread over the abdomen of the mice using a pipettor that delivers 50ug.

### DNA vaccines

Precipitation of DNA onto gold particles is achieved using standard procedures for the calcium/spermidine formulation of DNA vaccines. DNA is mixed with 2 micron gold particles in a small centrifuge tube containing 300 ml of 50 mM spermidine. The amount of DNA added is 2 ug per mg gold particles and typically batches of 26 mg gold (52 ug of DNA) were made. The DNA is precipitated onto gold by the addition of a 1/10 volume of 10% CaCl₂ during continuous agitation of the tube on a rotary mixer. DNA-gold complexes are washed three times with absolute ethanol then loaded into Tefzel tubing, dried and cut into 0.5 inch segments for use in the XR-1 device.

For immunization, DNA vaccines are delivered by the XR-1 device into the abdomen of Balb/C mice. A single shot is given for the immunization. Some experiments will employ a prime only, and others will have a prime and a boost at 4 weeks.

### Antibody ELISA

Serum samples are assayed for antibodies using an ELISA assay. Falcon Pro Bind microtiter plates are coated overnight at 4 °C with antigen in PBS (phosphate buffered saline, BioWhittaker). For HBsAg ELISA the antigen is purified HBsAg (BioDesign) at 0.1 ug per well. The plates are blocked for 1 hour at RT with 5% dry milk/PBS then washed 3X with wash buffer (10 mM Tris Buffered saline, 0.1% Brij-35) and serum samples diluted in dilution buffer (2% dry milk/PBS/0.05 % Tween 20) are added to the plate and then incubated for 2 hours at RT. Plates are washed 3X and a biotinylated goat anti-mouse antibody (Southern Biotechnology) diluted 1:8000 in dilution buffer is added to the plate and incubated for 1 hr at RT.

Following the incubation, plates are washed 3X, then a Streptavidin-Horseradish peroxidase conjugate (Southern Biotechnology) diluted 1:8000 in PBS is added and the plate incubated a further 1 hr at RT. Plates are washed 3X, then substrate solution added (BioRad) and the reaction is stopped with 1N H₂SO₄. Optical density is read at 450 nm. Endpoint titers for HBsAg are calculated by comparison of the samples with a standard of known titer. For subclass evaluation the biotinylated anti-mouse antibodies used are either IgG1 or IgG2a specific but the assay remains the same.

### Cell Culture

Single cell suspensions are obtained from mouse spleens. Spleens are squeezed through a mesh to produce a single cell suspension and cells are then sedimented, and treated with ACK buffer (Bio Whittaker, Walkersville MD) to lyse red blood cells. The cells are then washed twice in RPMI 1640 media supplemented with HEPES, 1 % glutamine (Bio Whittaker), and 5% heat inactivated fetal calf serum (FCS, Harlan, Indianapolis IN). Cells are counted, and resuspended to an appropriate concentration in "Total" media consisting of RPMI 1640 with HEPES and 1% glutamine, supplemented with 5% heat inactivated FCS, 50 mM mercaptoethanol (Gibco-BRL, Long Island NY), gentamycin (Gibco-BRL), 1 mM MEM sodium pyruvate (Gibco-BRL) and MEM non-essential amino acids (Sigma, St. Louis MO). For the CD8 specific assays cells are cultured *in vitro* in the presence of a peptide corresponding to a known CD8 epitopes. For HBsAg in BALB/C mice the sequence of the peptide is IPQSLDSWWTSL (QCB Inc). Peptides are made up in DMSO (10 mg/ml) and diluted to 10 mg/ml in culture medium.

### ELISPOTs

For IFN-g ELISPOTs assays Millipore Multiscreen membrane filtration plates are coated with 50 ul of 15 ug/ml anti-IFN-g antiserum (Pharmingen) in sterile 0.1M carbonate buffer pH 9.6, overnight at 4 °C. Plates are washed 6X with sterile PBS and then blocked with tissue culture medium containing 10% fetal bovine serum (FBS) for 1-2 hr at RT. The medium is removed and spleen cells dispensed into the wells with a total of 1X10⁶ cells per well. For wells in which less than 1X10⁶ cells from immunized animals is added, cells from naive animals are used to bring the total to 1X10⁶. Cells are incubated overnight in a tissue culture incubator in the presence of the peptide as described above. Plates are washed 2X with PBS and 1X with distilled water. This is followed with 3 washes with PBS. Biotinylated anti IFN-g monoclonal antibody (Pharmingen) is added to the plate (50 ul of a 1 ug/ml solution in PBS) and incubated for 2 hr at RT. Plates are washed 6X with PBS then 50 ul of a Streptavidin Alkaline phosphatase conjugate (1:1000 in PBS, Pharmingen) is added and incubated for 2 hr at RT. Plates are washed 6X with PBS and the color substrate (BioRad) is added and the reaction is allowed to proceed until dark spots appear. The reaction is stopped by washing with water 3X. Plates are air dried and spots counted under a microscope.

## Claims

1. A compound for use in a method for enhancing or stimulating in an individual a cell-mediated immune response to an antigen, wherein the compound is an imidazoquinoline amine, imidazopyridine amine, 6,7-fused cycloalkylimidazopyridine amine, 1,2-bridged imidazoquinoline amine, thiazolo- or oxazolo-quinolinamine, thiazolo- or oxazolo-pyridinamine, imidazonaphthyridine or tetrahydroimidazonaphthyridine amine, and wherein the compound is administered topically or transdermally to the individual 12 to 36 hours after an isolated recombinant polynucleotide capable of expressing the antigen is administered.

2. A compound for use according to claim 1, wherein the compound is an imidazoquinoline.

3. A compound for use according to claim 1 or 2, wherein the compound is imiquimod.

4. A compound for use according to any one of the preceding claims, wherein the polynucleotide is administered topically or transdermally.

5. A compound for use according to any one of the preceding claims, wherein the compound and/or polynucleotide is/are administered in the form of particles.

6. A compound for use according to claim 4 or 5, wherein the polynucleotide and/or compound is coated on a core carrier.

7. A compound for use according to claim 6, wherein the core carrier is constituted of gold particles.

8. A compound for use according to any one of claims 5 to 7, wherein the particles are administered using a needleless syringe.

9. A compound for use according to any one of claims 1 to 4, in which the compound is administered in the form of a cream

10. A compound for use according to any one of the preceding claims, wherein the polynucleotide is administered repeatedly to provide a prime and booster administration.

11. A compound for use according to any one of the preceding claims, wherein the polynucleotide enhances or stimulates an immune response against a viral infection.

12. A compound for use according to claim 11, wherein the virus is hepatitis B virus, herpes simplex virus, human immunodeficiency virus, influenza virus or papillomavirus.

13. A compound for use according to any one of the preceding claims, wherein the polynucleotide is DNA.

14. An isolated recombinant polynucleotide capable of expressing an antigen for use in a method for enhancing or stimulating in an individual a cell-mediated immune response to said antigen, wherein the polynucleotide is administered 12 to 36 hours before topical or transdermal administration to the individual of a compound that is an imidazoquinoline amine, imidazopyridine amine, 6,7-fused cycloalkylimidazopyridine amine, 1,2-bridged imidazoquinoline amine, thiazolo- or oxazolo-quinolinamine, thiazolo- or oxazolo-pyridinamine, imidazonaphthyridine or tetrahydroimidazonaphthyridine amine.

15. Use of a compound in the manufacture of a medicament for enhancing or stimulating in an individual a cell-mediated immune response to an antigen, wherein the compound is an imidazoquinoline amine, imidazopyridine amine, 6,7-fused cycloalkylimidazopyridine amine, 1,2-bridged imidazoquinoline amine, thiazolo- or oxazolo-quinolinamine, thiazolo- or oxazolo-pyridineamine, imidazonaphthyridine or tetrahydroimidazonaphthyridine amine, and wherein the compound is for administration topically or transdermally to the individual 12 to 36 hours after an isolated recombinant polynucleotide capable of expressing the antigen is administered.

## Patentansprüche

1. Verbindung zur Verwendung bei einer Methode zum Verstärken oder Stimulieren in einem Individuum einer Zell-vermittelten Immunantwort auf ein Antigen, wobei die Verbindung ein Imidazochinolinamin, Imidazopyridinamin, 6,7-fusioniertes Cycloalkylimidazopyridinamin, 1,2-überbrücktes Imidazochinolinamin, Thiazolo- oder Oxazolo-Chinolinamin, Thiazolo- oder Oxazolo-Pyridinamin, Imidazonaphthyridin oder Tetrahydroimidazonaphthyridinamin ist, und wobei die Verbindung an das Individuum 12 bis 36 Stunden nach Verabreichung eines zum Exprimieren des Antigens fähigen isolierten rekombinanten Polynukleotids topisch oder transdermal verabreicht wird.

2. Verbindung zur Verwendung nach Anspruch 1, wobei die Verbindung ein Imidazochinolin ist.

3. Verbindung zur Verwendung nach Anspruch 1 oder 2, wobei die Verbindung Imiquimod ist.

4. Verbindung zur Verwendung nach jedem der vorangehenden Ansprüche, wobei das Polynukleotid topisch oder transdermal verabreicht wird.

5. Verbindung zur Verwendung nach jedem der vorangehenden Ansprüche, wobei die Verbindung und/oder das Polynukleotid in Form von Partikeln verabreicht wird/werden.

6. Verbindung zur Verwendung nach Anspruch 4 oder 5, wobei das Polynukleotid und/oder die Verbindung auf einen Core-Träger aufgeschichtet wird.

7. Verbindung zur Verwendung nach Anspruch 6, wobei der Core-Träger aus Goldpartikeln besteht.

8. Verbindung zur Verwendung nach jedem der Ansprüche 5 bis 7, wobei die Partikel unter Verwendung einer nadellosen Spritze verabreicht werden.

9. Verbindung zur Verwendung nach jedem der Ansprüche 1 bis 4, wobei die Verbindung in Form einer Creme verabreicht wird.

10. Verbindung zur Verwendung nach jedem der vorangehenden Ansprüche, wobei das Polynukleotid wiederholt verabreicht wird, um für eine Prime- und Booster-Verabreichung (Aktivierung und Auffrischung) zu sorgen.

11. Verbindung zur Verwendung nach jedem der vorangehenden Ansprüche, wobei das Polynukleotid eine Immunantwort gegen eine virale Infektion verstärkt oder stimuliert.

12. Verbindung zur Verwendung nach Anspruch 11, wobei das Virus Hepatitis B-Virus, Herpes simplex-Virus, humanes Immunschwäche-Virus, Influenza-Virus oder Papillomavirus ist.

13. Verbindung zur Verwendung nach jedem der vorangehenden Ansprüche, wobei das Polynukleotid DNA ist.

14. Isoliertes rekombinantes Polynukleotid, das zum Exprimieren eines Antigens fähig ist, zur Verwendung bei einer Methode zum Verstärken oder Stimulieren in einem Individuum einer Zell-vermittelten Immunantwort auf dieses Antigen, wobei das Polynukleotid 12 bis 36 Stunden vor der topischen oder transdermalen Verabreichung an das Individuum einer Verbindung, die ein Imidazochinolinamin, Imidazopyridinamin, 6,7-fusioniertes Cycloalkylimidazopyridinamin, 1,2-überbrücktes Imidazochinolinamin, Thiazolo- oder Oxazolo-Chinolinamin, Thiazolo- oder Oxazolo-Pyridinamin, Imidazonaphthyridin oder Tetrahydroimidazonaphthyridinamin ist, verabreicht wird.

15. Verwendung einer Verbindung in der Herstellung eines Medikaments zum Verstärken oder Stimulieren in einem Individuum einer Zell-vermittelten Immunantwort auf ein Antigen, wobei die Verbindung ein Imidazochinolinamin, Imidazopyridinamin, 6,7-fusioniertes Cycloalkylimidazopyridinamin, 1,2-überbrücktes Imidazochinolinamin, Thiazolo- oder Oxazolo-Chinolinamin, Thiazolo- oder Oxazolo-Pyridinamin, Imidazonaphthyridin oder Tetrahydroimidazonaphthyridinamin ist, und wobei die Verbindung an das Individuum 12 bis 36 Stunden nach Verabreichung eines zum Exprimieren des Antigens fähigen isolierten rekombinanten Polynukleotids topisch oder transdermal verabreicht wird.

## Revendications

1. Composé pour utilisation dans une méthode destinée à accroître ou à stimuler chez un individu une réponse immunitaire à médiation cellulaire contre un antigène, dans lequel le composé est une imidazoquinoline-amine, imidazopyridine-amine, cycloalkylimidazopyridine-amine fusionnée en 6,7, imidazoquinoline-amine pontée en 1,2, thiazolo- ou oxazolo-quinolinamine, thiazolo- ou oxazolo-pyridinamine, imidazonaphthyridine ou tétrahydroimidazonaphthyridine-amine, et dans lequel le composé est administré de manière topique ou transdermique à l'individu 12 à 36 heures après avoir administré un polynucléotide recombinant isolé capable d'exprimer l'antigène.

2. Composé utilisable selon la revendication 1, dans lequel le composé est une imidazoquinoline.

3. Composé utilisable selon la revendication 1 ou 2, dans lequel le composé est l'imiquimod.

4. Composé utilisable selon l'une quelconque des revendications précédentes, dans lequel le polynucléotide est administré de manière topique ou transdermique.

5. Composé utilisable selon l'une quelconque des revendications précédentes, dans lequel le composé et/ou le polynucléotide est/sont administré(s) sous forme de particules.

6. Composé utilisable selon la revendication 4 ou 5, dans lequel le polynucléotide et/ou le composé est enrobé sur un véhicule noyau.

7. Composé utilisable selon la revendication 6, dans lequel le véhicule noyau est constitué de particules d'or.

8. Composé utilisable selon l'une quelconque des revendications 5 à 7, dans lequel les particules sont administrées en utilisant une seringue sans aiguille.

9. Composé utilisable selon l'une quelconque des revendications 1 à 4, dans lequel le composé est administré sous forme d'une crème.

10. Componé utilisable selon l'une quelconque des revendications précédentes, dans lequel le polynucléotide est administré de manière répétée pour fournir une première administration et une administration de rappel.

11. Composé utilisable selon l'une quelconque des revendications précédentes, dans lequel le polynucléotide accroît ou stimule une réponse immunitaire contre une infection virale.

12. Composé utilisable selon la revendication 11, dans lequel le virus est le virus de l'hépatite B, le virus de l'herpes, le virus de l'immunodéficience humaine, le virus de la grippe ou un papillomavirus.

13. Componé utilisable selon l'une quelconque des revendications précédentes, dans lequel le polynucléotide est un ADN.

14. Polynucléotide recombinant isolé capable d'exprimer un antigène utilisable dans une méthode destinée à accroître ou à stimuler chez un individu une réponse immunitaire à médiation cellulaire contre ledit antigène, dans lequel le polynucléotide est administré 12 à 36 heures avant l'administration topique ou transdermique à l'individu d'un composé qui est une imidazoquinoline-amine, imidazopyridine-amine, cycloalkylimidazopyridine-amine fusionnée en 6,7, imidazoquinoline-amine pontée en 1,2, thiazolo- ou oxazolo-quinolinamine, thiazolo- ou oxazolo-pyridinamine, imidazonaphthyridine ou tétrahydroimidazonaphthyridine-amine.

15. Utilisation d'un composé dans la préparation d'un médicament destiné à accroître ou à stimuler chez un individu une réponse immunitaire à médiation cellulaire à un antigène, dans laquelle le composé est une imidazoquinoline-amine, imidazopyridine-amine, cycloalkylimidazopyridine-amine fusionnée en 6,7, imidazoquinoline-amine pontée en 1,2, thiazolo- ou oxazolo-quinolinamine, thiazolo- ou oxazolo-pyridinamine, imidazonaphthyridine ou tétrahydroimidazonaphthyridine-amine, et dans laquelle le composé est administré de manière topique ou transdermique à l'individu 12 à 36 heures après avoir administré un polynucléotide recombinant isolé capable d'exprimer l'antigène.
